# EUROPÄISCHE PATENTSCHRIFT

(11) **EP 2 313 406 B1**
(45) Veröffentlichungstag und Bekanntmachung des Hinweises auf die Patenterteilung: **25.02.2015**
(21) Anmeldenummer: 09780981.8
(22) Anmeldetag: 23.07.2009
(51) Int. Cl.: C07D 471/04, C07D 519/00, A61K 31/4375, A61P 11/06

(54) **4-DIMETHYLAMINOPHENYL-SUBSTITUIERTE NAPTHTHYRIDINE UND IHRE VERWENDUNG ALS ARZNEIMITTEL**
4-DIMETHYLAMINOPHENYL SUBSTITUTED NAPTHTHYRIDINES UND THEIR USE AS MEDICINE
NAPTHTHYRIDINES SUBSTITUÉES PAR 4-DIMÉTHYLAMINOPHÉNYLE ET LEUR UTILISATION EN TANT QUE MÉDICAMENT

(30) Priorität: 05.08.2008 EP 08161843
(43) Veröffentlichungstag der Anmeldung: 27.04.2011
(73) Patentinhaber: Boehringer Ingelheim International GmbH, 55216 Ingelheim am Rhein (DE)
(72) Erfinder: FIEGEN, Dennis, 55216 Ingelheim Am Rhein (DE); HANDSCHUH, Sandra, 55216 Ingelheim Am Rhein (DE); HOBBIE, Silke, 55216 Ingelheim Am Rhein (DE); HOFFMANN, Matthias, 55216 Ingelheim Am Rhein (DE); KONO, Takeshi, 55216 Ingelheim Am Rhein (DE); SATO, Yayoi, 55216 Ingelheim Am Rhein (DE); SCHNAPP, Andreas, 55216 Ingelheim Am Rhein (DE); SCHULER-METZ, Annette, 55216 Ingelheim Am Rhein (DE)
(74) Vertreter: Simon, Elke Anna Maria
(86) Internationale Anmeldenummer: PCT/EP2009/059493
(87) Internationale Veröffentlichungsnummer: WO 2010/015518

(56) Entgegenhaltungen:
- WO-A-03/057695

## Beschreibung

Die Erfindung betrifft neue substituierte Naphthyridine der Formel **1**, sowie pharmakologisch verträgliche Salze, Diastereomere, Enantiomere, Racemate, Hydrate oder Solvate davon, 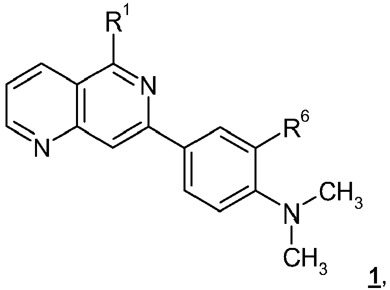 wobei
**R¹** ein Rest **A** ausgewählt aus der Gruppe bestehend aus -O-R³, -NR³R⁴, wobei **R³, R⁴, R⁶** und **m** die in Anspruch 1 beschriebenen Bedeutungen haben können, sowie pharmazeutische Zusammensetzungen, die diese Verbindungen enthalten.

### 1. HINTERGRUND DER ERFINDUNG

### 1.1 SYK-Inhibitoren

Die vorliegende Erfindung beschreibt neue substituierte Naphthyridine, welche die Proteinkinase Syk (**s**pielen **t**yrosine **k**inase) hemmen, deren Herstellung und Formulierung sowie ihre Verwendung zur Herstellung eines Arzneimittels.

Syk ist eine intrazelluläre Tyrosin Kinase, die eine wichtige Mediatorfunktion in der Signaltransduktion verschiedener Rezeptoren in B-Zellen, Mastzellen, Monozyten, Makrophagen, Neutrophilen, T-Zellen, dendritischen Zellen und Epithelzellen hat. Zu den Rezeptoren, bei denen Syk eine wichtige Funktion in der Signaltransduktion spielt, gehören z.B. die Rezeptoren für IgE (FcεRI) und IgG (FcγR1) auf Mastzellen und B-Zellen, der B-Zell Rezeptor (BCR) und der T-Zell Rezeptor (TCR) auf B- und T-Zellen, der ICAM1 Rezeptor (ICAM1 R) auf Epithelzellen der Atemwege, der DAP12-Rezeptor auf natürlichen Killer-Zellen, dendritischen Zellen und Osteoklasten, der Dectin 1-Rezeptor auf einer Subpopulation von T-Helfer Zellen (Th-17 Zellen), sowie die Integrin Rezeptoren für β1-, β2,- und β3-Integrine auf Neutrophilen, Monozyten und Makrophagen (Wong et al.; Expert Opin. Investig. Drugs (2004) 13(7), 743-762; Ulanova et al.; Expert Opion. Ther. Target (2005) 9(5); 901-921; Wang et al.; J. Immunol. (2006) 177, 6859-6870; LeibundGut-Landmann et al.; Nature Immunology (2007) 8, 630-638; Slack et al., European J. Immunol. (2007) 37, 1600-1612). Am besten beschrieben sind die molekularen Vorgänge während der Signaltransduktion des FcεRI. In Mastzellen bewirkt das Binden von IgE an den FcεRI die Vernetzung (cross-linking) von IgE-Rezeptoren und die Rekrutierung und Aktivierung von Lyn (einer Tyrosinkinase aus der Src Familie). Aktives Lyn phoshoryliert so genannte ITAM-Motive, die in vielen der oben aufgezählten Rezeptoren vorhanden sind, und generiert dadurch Bindungsstellen für die SH2-Domäne von Syk. Als Ergebnis der Bindung an das ITAM -Motiv wird Syk aktiviert und phosphoryliert daraufhin verschiedene Substrate, die für die Freisetzung von allergischen und entzündlichen Mediatoren wie z.B. Histamin und β-Hexosamidase (βHA), sowie für die Synthese von Lipid-Mediatoren, wie z.B. Prostaglandine und Leukotriene, benötigt werden. Auf Grund seiner zentralen Funktion in verschiedenen Signaltransduktionswegen wird Syk als therapeutisches Target für verschiedene Erkrankungen wie z.B. allergische Rhinitis, Asthma, Autoimmun-Erkrankungen, rheumatoide Arthritis, Osteopenie, Osteoporose, COPD, sowie verschiedene Leukämien und Lymphome diskutiert (Wong et al.; Expert Opin. Investig. Drugs (2004) 13(7), 743-762; Ulanova et al.; Expert Opion. Ther. Target (2005) 9(5); 901-921; Sigh und Masuda. Annual Reports in Medicinal Chemistry (2007) Vol 42; 379-391; Bajpai et al.; Expert Opin. Investig. Drugs (2008) Vol 15 (5); 641-659; Masuda and Schmitz; PPT (2008) Vol 21; 461-467).

Allergische Rhinitis und Asthma sind Erkrankungen, die mit allergischen Reaktionen und Entzündungsvorgängen assoziiert sind und an denen verschiedene Zelltypen beteiligt sind wie z.B. Mastzellen, Eosinophile, T-Zellen und dendritische Zellen. Nach einer erfolgten Exposition mit Allergenen werden die hoch-affinen Immunglobulin-Rezeptoren für IgE (FcεRI) und IgG (FcγR1) aktiviert und induzieren die Freisetzung vom pro-entzündlichen Mediatoren und Bronchokonstriktoren. Ein Inhibitor der Syk Kinase Aktivität sollte somit diese Schritte hemmen können.

Rheumatoide Arthritis (RA) ist eine Autoimmun-Erkrankung, in der die Gelenke umgebenen Knochen und Knorpelstrukturen progressiv zerstört werden. In der Pathophysiologie von RA spielen B-Zellen eine signifikante Rolle, was z.B. durch den therapeutischen Nutzen von Rituximab, einem B Zell-depletierenden Antikörper gezeigt wurde. Neben der Funktion von Syk in der Signaltransduktion des BCR (der nach seiner Stimulation ebenfalls die Feisetzung von pro-Entzündlichen Mediatoren induziert, spielt Syk auch bei der Reifung und der Proliferation von B-Zellen eine wichtige Rolle (Cheng et al. Nature (1995) 378, 303-306, Cornall et al., PNAS (2000) 97(4), 1713-1718). Ein Inhibitor der Syk -Kinaseaktivität könnte somit eine therapeutische Option für die Behandlung von Autoimmun-Erkrankungen wie RA und Erkrankungen mit einer erhöhten Proliferation von B-Zellen, wie z.B. B-Zell-Lymphozyten bieten.

Die chronisch obstruktive Lungenerkrankung (COPD) ist gekennzeichnet durch eine sukzessive Verschlechterung der Lungenfunktion und einer chronischen Entzündung der Atemwege, die durch Noxen verschiedenster Art initiiert und erhalten wird und zur Aufrechterhaltung des Krankheitsgeschehens beiträgt. Auf zellulärer Ebene findet sich bei der COPD vor allem eine Vermehrung von T-Lymphozyten, Neutrophilen, Granulozyten und Makrophagen. Insbesondere die Zahl der CD8-positiven Lymphozyten ist erhöht, die in direkte Verbindung mit der Verschlechterung der Lungenfunktion gebracht wird. Ein weiteres Charakteristikum der COPD sind akute Verschlechterungen der Lungenfunktion (Exazerbationen), die durch virale (z.B. Rhinovirus), oder bakterielle (z.B. Streptococcus pneumoniae, Haemophilus influenzae und Moraxella catarrhalis) Infektionen gekennzeichnet sind.

Aufgrund der beschriebenen pro-entzündlichen Funktion von Syk in Makrophagen, T-Zellen und Neutrophilen (siehe: Wong et al.; Expert Opin. Investig. Drugs (2004) 13(7), 743-762; und Zitate darin) könnte ein Inhibitor der Syk-Kinase-Aktivität ein neuer therapeutischer Ansatz für die Behandlung der der COPD zugrunde liegenden Entzündungsvorgänge sein. Darüber hinaus wurde gezeigt, dass Syk in Atemwegs-Epithelzellen an der durch den ICAM1 R vermittelten Aufnahme und nachfolgender Replikation des Rhinovirus beteiligt ist und dass eine si-RNA gegen Syk diese Schritte blockiert (Wang et al.; J. Immunol. (2006) 177, 6859-6870; Lau et al.; J. Immunol. (2008) 180, 870-880). Somit könnte ein Inhibitor der Syk-Kinase-Aktivität auch bei Exazerbationen, die durch Rhinoviren verursacht werden, therapeutisch genutzt werden.

Verschiedene Studien implizieren, dass Syk an der malignen Transformation von Lymphozyten beteiligt ist (Zusammengefasst in Sigh und Masuda. Annual Reports in Medicinal Chemistry (2007) Vol 42; 379-391). Ein TEL-Syk Fusionsprotein mit konstitutiver Syk-Aktivität transformierte B-Zellen eines Patienten mit myelodysplastischen Syndrom, ein konstitutiv aktives ITK-Syk Fusionsprotein wurde aus Patienten mit T-Zell-Lymphomen isoliert. Weiterhin wurde konstitutiv aktives Syk in B-Zell Lymphom Zellen von Patienten gefunden. Basierend auf diesen Daten erscheint es, dass Syk ein Proto-Onkogen in hematopoietischen Zellen ist und ein potentielles Target für die Behandlung von bestimmten Leukämien und Lymphomen darstellt.

### 1.2 Stand der Technik

BE 835770 beschreibt 5-Amino-1,6-Naphthyridine mit antimikrobieller Aktivität. US Patent-Nr. US 3,928,367, US 4,017,500, US 4,115,395 und US 4,260,759 beschreiben 5-Amino-1,6-Naphthyridine mit antifungaler und antibakterieller Aktivität. WO 9918077 beschreiben 5-piperazinyl-1,6-naphthyridine als Serotonin-Antagonisten. US Patent US 7,321,041 beschreibt substituierte [1,6]-Naphthyridine als SYK-Inhibitoren, die jedoch ein zu den erfindungsgemäßen Verbindungen völlig verschiedenes Substitutionsmuster aufweisen. Weitere Naphthyridine als SYK-Inhibitoren sind aus WO 03/057695 bekannt.

### 2. BESCHREIBUNG DER ERFINDUNG

Die Erfindung ist in den Patentansprüchen definiert. Die folgende Beschreibung unterliegt dieser Einschränkung. Gegenstände sowie Beispiele der Beschreibung, die nicht unter den Umfang der Ansprüche fallen, sind nur Teil der Offenbarung aber nicht der beanspruchten Erfindung.

Überraschenderweise konnte nun gefunden werden, dass Naphthyridine der Formel **1** besonders geeignet sind zur Behandlung von Atemwegserkrankungen, allergischen Erkrankungen, Osteoporose, gastrointestinalen Erkrankungen, Autoimmunerkrankungen, entzündliche Erkrankungen sowie Erkrankungen des periphären oder zentralen Nervensystems, insbesondere zur Behandlung von Asthma, allergische Rhinitis, rheumatoide Arthritis, allergische Dermatitis und COPD.
Gegenstand der vorliegenden Erfindung sind deshalb Verbindungen der Formel **1**, 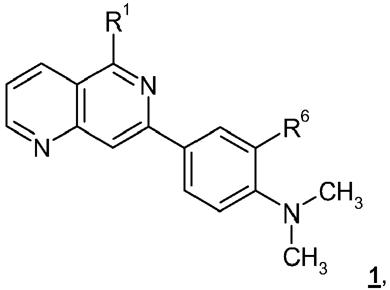 wobei
**R¹** ein Rest **A** ausgewählt ist aus der Gruppe bestehend aus -O-R³, -NR³R⁴,
**R³** ein Rest ist ausgewählt aus der Gruppe bestehend aus -C₁₋₆-Alkyl, -C₁₋₆-Fluoroalkyl, -(C₁₋₅-Alkyl)-OH, -C₆₋₁₀-Aryl, -(C₁₋₄-Alkylen)-(C₆₋₁₀-Aryl), -Ethenyl, -(C₁₋₄-Alkylen)-(Ethen), -Ethinyl, - (C₁₋₄-Alkylen)-(Ethin), -(C₁₋₄-Alkylen)-(Ethin)-NH₂, -(C₁₋₄-Alkylen)-(Ethin)-(C₁₋₄-Alkylen)-NH₂, - NH(C₁₋₃-Alkyl), -(C₁₋₄-Alkylen)-NH(C₁₋₃-Alkyl), -CHOH-(C₁₋₄-Alkylen)-NH₂, -(C₁₋₄-Alkylen)-CHOH-(C₁₋₄-Alkylen)-NH₂, -(C₁₋₄-Alkylen)-CHOH-NH₂, -CHOH-NH₂, mono- oder bicyclisches, gesättigtes oder teilweise gesättigtes -C₃₋₁₀-Cycloalkyl, mono- oder bicyclisches, gesättigtes oder teilweise gesättigtes -(C₁₋₄-Alkylen)-C₃₋₁₀-Cycloalkyl, -(Het), -(C₁₋₄-Alkylen)-(Het), -(Hetaryl), und -(C₁₋₄-Alkylen)-(Hetaryl),
wobei dieser Rest
gegebenenfalls mit einem oder mehreren Resten unabhängig voneinander ausgewählt aus der Gruppe bestehend aus H, -OH, -Oxo, -COOH, -Halogen, -C₁₋₃-Alkyl, -C₁₋₃-Haloalkyl, -C₁₋₃-Alkyl-OH, -C₃₋₇Cycloalkyl, -O-(C₁₋₄-Alkyl), -NH(C₁₋₄-Alkyl), -(C₁₋₄-Alkylen)-NH(C₁₋₄-Alkyl), -N(C₁₋₄-Alkyl)₂, -(C₁₋₄-Alkylen)-N(C₁₋₄-Alkyl)₂, -NH-CO-NH₂, -(C₁₋₄-Alkylen)-NH-CO-NH₂, -CO-NH₂, -(C₁₋₄-Alkylen)-CO-NH₂, -CO-NH(C₁₋₃-Alkyl), -(C₁₋₄-Alkylen)-CO-NH(C₁₋₃-Alkyl), -CO-N(C₁₋₃-Alkyl)₂, -(C₁₋₄-Alkylen)-CO-N(C₁₋₃-Alkyl)₂, -NH-(CO)ₘ-NH₂, -NH-(C₁₋₄-Alkylen)-(CO)ₘ-NH₂, -NH-(CO)ₘ-NH(C₁₋₃-Alkyl), -NH-(C₁₋₄-Alkylen)-(CO)ₘ-NH(C₁₋₃-Alkyl), -NH-(CO)ₘ-N(C₁₋₃-Alkyl)₂, -NH-(C₁₋₄-Alkylen)-(CO)ₘ-N(C₁₋₃-Alkyl)₂, -O-(C₂₋₄-Alkylen)-NH₂, -O-(C₂₋₄-Alkylen)-NH(C₁₋₃-Alkyl), -O-(C₂₋₄-Alkylen)-N(C₁₋₃-Alkyl)₂, -NH-CO-(C₁₋₃-Alkyl), -(C₁₋₄-Alkylen)-NH-CO-(C₁₋₃-Alkyl), C₃₋₅-Cycloalkyl, -SO₂-(C₁₋₄-Alkyl), -SO₂-(C₃₋₅-Cycloalkyl), -SO₂-NH₂, -SO₂-NH-C₁₋₃-Alkyl, -SO₂-N(C₁₋₃-Alkyl)₂, -SO₂-(Het), -O-(Het), -O-(C₁₋₄-Alkylen)-(Het), -NH-(Het), -NH-(C₁₋₄-Alkylen)-(Het), -NH-(Hetaryl), -NH-(C₁₋₄-Alkylen)-(Hetaryl), - (Het) und -(C₁₋₄-Alkylen)-(Het) substituiert sein kann,
wobei **(Het)** für einen drei- bis zehngliedrigen, gesättigten oder teilweise gesättigten, mono- oder bicyclischen, gegebenenfalls mit 1-3 Resten ausgewählt aus C₁₋₃-Alkyl, Halogen, CH₂-NH₂, NH₂, OH, CO-NH₂ und Oxo substituierten Heterocyclus steht, der 1, 2 oder 3 Heteroatome unabhängig voneinander ausgewählt aus der Gruppe bestehend aus N, O und S enthält,
und
wobei **(Hetaryl)** für ein fünf- bis zehngliedriges, mono- oder bicyclisches, gegebenenfalls mit 1, 2 oder 3 Resten ausgewählt aus C₁₋₃-Alkyl, Halogen, CH₂-NH₂, NH₂, OH, CO-NH₂ und Oxo substituiertes Heteroaryl steht, das 1-3 Heteroatome unabhängig voneinander ausgewählt aus der Gruppe bestehend aus N, O und S enthält,
wobei
**m** = 0 oder 1
und
R⁴ H, Methyl oder Ethyl
   und
**R⁶** Methyl bedeuten, sowie pharmazeutisch verträgliche Salze davon.

Die vorliegende Erfindung betrifft weiterhin bevorzugt Verbindungen der Formel **1** mit den vorstehend genannten Bedeutungen der einzelnen Variablen,
wobei R¹ -O-R³ oder -NR³R⁴ ist
und wobei
**(Het)** für einen drei- bis siebengliedrigen, gesättigten oder teilweise gesättigten, monocyclischen, gegebenenfalls mit 1-3 Resten ausgewählt aus Methyl, Ethyl Propyl, Isopropyl, F, Cl, Br, CH₂-NH₂, NH₂, OH, CO-NH₂ und Oxo substituierten Heterocyclus steht, der 1, 2 oder 3 Heteroatome unabhängig voneinander ausgewählt aus der Gruppe bestehend aus N, O und S enthält, und wobei
**(Hetaryl)** für ein fünf- bis sechsgliedriges, monocyclisches, gegebenenfalls mit 1, 2 oder 3 Resten ausgewählt aus Methyl, Ethyl Propyl, Isopropyl, F, Cl, Br, CH₂-NH₂, NH₂, OH, CO-NH₂ und Oxo substituiertes Heteroaryl steht, das 1-3 Heteroatome unabhängig voneinander ausgewählt aus der Gruppe bestehend aus N, O und S enthält,
sowie pharmazeutisch verträgliche Salze, Diastereomere, Enantiomere, Racemate, Hydrate und Solvate davon.

Insbesondere betrifft die vorliegende Erfindung Verbindungen der Formel **1** mit den vorstehend genannten Bedeutungen der einzelnen Variablen,
wobei R¹ ausgewählt ist aus der Gruppe bestehend aus 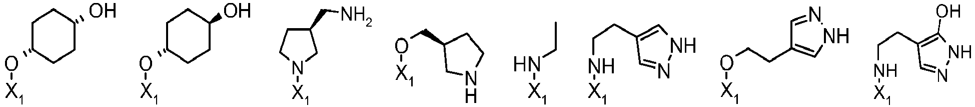 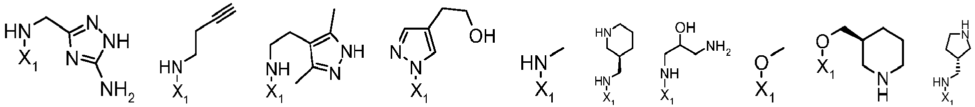 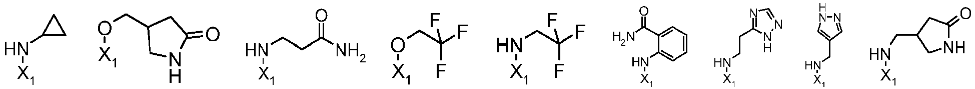 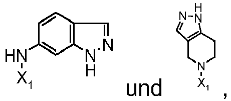 wobei X₁ den Anknüpfungspunkt von R¹ an die Struktur der Formel **1** bezeichnet und und wobei R⁶ Methyl ist.
sowie pharmazeutisch verträgliche Salze davon.

Ein weiterer Gegenstand der Erfindung sind die obigen Verbindungen der Formel **1** mit den vorstehend genannten Bedeutungen der einzelnen Variablen als Arzneimittel.

Ein weiterer Gegenstand der Erfindung ist die Verwendung der obigen Verbindungen der Formel **1** mit den vorstehend genannten Bedeutungen der einzelnen Variablen zur Herstellung eines Medikaments zur Behandlung von Erkrankungen, die sich durch die Inhibition des SYK-Enzyms behandeln lassen.

Ein weiterer bevorzugter Gegenstand der Erfindung ist die Verwendung der obigen Verbindungen der Formel **1** mit den vorstehend genannten Bedeutungen der einzelnen Variablen zur

Herstellung eines Medikaments zur Behandlung von Erkrankungen ausgewählt aus der Gruppe bestehend aus Allergische Rhinitis, Asthma, COPD, Adultes respiratorisches Distress Syndrom, Bronchitis, Dermatitis und Kontakt Dermatitis, allergische Dermatitis, allergische Rhinokonjunktivitis, rheumatoide Arthritis, Anti-Phospholipid Syndrom, Berger-Krankheit, Evans Syndrom, Kolitis ulcerosa, allergische Antikörper-basierende Glomerulonephritis, Granulozytopenie, Goodpasture Syndrom, Hepatitis, Henoch-Schönlein Purpura, Hypersensitivitätsvaskulitis, Immunhämolytische Anämie, idiopathische thrombozytopenische Purpura, Kawasaki Syndrom, Allergische Konjunktivitis, Lupus erythematodes, Neutropenie, Nicht familiäre lateral Sklerose, Morbus Crohn, multiple Sklerose, Myastenia Gravis, Osteoporose, Osteolytische Erkrankungen,Osteopenie, Psiorasis, Sjögren Syndrome, Sklerodermie, Urtikaria / Angioödema, Wegener-Granulomatose und Zöliakie.
Ein weiterer besonders bevorzugter Gegenstand der vorliegenden Erfindung ist die Verwendung der obigen Verbindungen der Formel **1** mit den vorstehend genannten Bedeutungen der einzelnen Variablen zur Herstellung eines Medikaments zur Behandlung von Erkrankungen ausgewählt aus der Gruppe bestehend aus Asthma, COPD, allergische Rhinitis, Adultes respiratorisches Distress Syndrom, Bronchitis, allergische Dermatitis, Kontakt Dermatitis, idiopathische thrombozytopenische Purpura, rheumatoide Arthritis und allergische Rhinokonjunktivitis.

Insbesondere betrifft die vorliegende Erfindung die Verwendung der obigen Verbindungen der Formel **1** mit den vorstehend genannten Bedeutungen der einzelnen Variablen zur Herstellung eines Medikaments zur Behandlung von Erkrankungen ausgewählt aus der Gruppe bestehend aus Asthma, COPD, allergische Rhinitis, allergische Dermatitis und rheumatoide Arthritis.

Weiterhin betrifft die vorliegende Erfindung vorzugsweise pharmazeutische Formulierungen, die einen Gehalt an einer oder mehreren Verbindungen der Formel **1** mit den vorstehend genannten Bedeutungen der einzelnen Variablen haben.

Ein weiterer Gegenstand der Erfindung sind pharmazeutische Formulierungen, die einen Gehalt an einer oder mehreren Verbindungen der Formel **1** mit den vorstehend genannten Bedeutungen der einzelnen Variablen haben, in Kombination mit einem Wirkstoff ausgewählt aus der Gruppe bestehend aus Betamimetika, Corticosteroiden, PDE4-Inhibitoren, EGFR-Hemmern und LTD4-Antagonisten, CCR3-Inhibitoren, iNOS-Inhibitoren und weiterer SYK-Inhibitoren.

Einen weiteren bevorzugten Gegenstand der Erfindung stellen die folgenden Zwischenprodukte bei der Herstellung der obigen Verbindungen nach Formel **1** ausgewählt aus der Gruppe bestehend aus 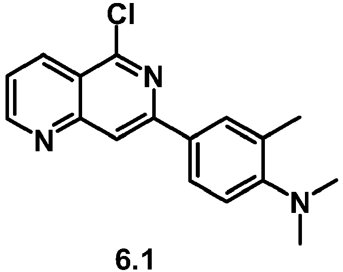 und 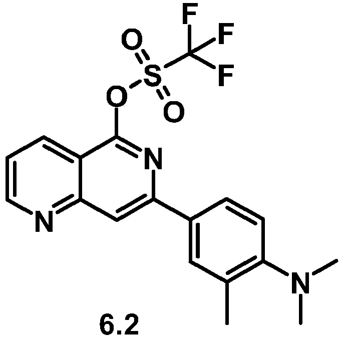 dar, sowie pharmazeutisch verträgliche Salze, Diastereomere, Enantiomere, Racemate, Hydrate und Solvate davon..

### 3. VERWENDETE BEGRIFFE UND DEFINITIONEN

Soweit nicht anders angegeben, sind alle Substituenten voneinander unabhängig. Sollten an einer Gruppe beispielsweise mehrere C₁₋₆-Alkylgruppen als Substituenten möglich sein, so könnte, beispielsweise im Fall von drei Substituenten C₁₋₆-Alkyl unabhängig voneinander beispielsweise einmal Methyl, einmal *n*-Propyl und einmal *tert*-Butyl bedeuten.

Im Rahmen dieser Anmeldung können bei der Definition von möglichen Substituenten, diese auch in Form einer Strukturformel dargestellt werden. Dabei wird ein Stern (*) in der Strukturformel des Substituenten als der Verknüpfungspunkt zum Rest des Moleküls verstanden. Weiterhin wird das auf den Verknüpfungspunkt folgende Atom des Substituenten als das Atom mit der Positionsnummer 1 verstanden. So werden zum Beispiel die Reste N-Piperidinyl **(I),** 4-Piperidinyl (II), 2-Tolyl (III), 3-Tolyl (IV) und 4-Tolyl (V) wie folgt dargestellt:

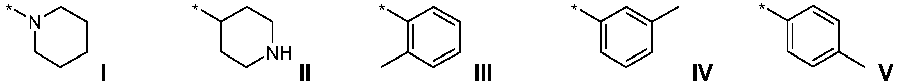

Befindet sich in der Strukturformel des Substituenten kein Stern (*), so kann an dem Substituenten jedes Wasserstoffatom entfernt werden und die dadurch frei werdende Valenz als Bindungsstelle zum Rest eines Molekül dienen. So kann zum Beispiel **VI** 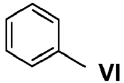 die Bedeutung von 2-Tolyl, 3-Tolyl, 4-Tolyl und Benzyl haben.
Alternativ zum * wird im Rahmen dieser Anmeldung auch X₁ als Anknüpfungspunkt des Restes R¹ zur Struktur der Formel **1** und X₂ als Anknüpfungspunkt des Restes R² zur Struktur der Formel **1** verstanden.

Unter dem Begriff "C₁₋₆-Alkyl" (auch soweit sie Bestandteil anderer Reste sind) werden verzweigte und unverzweigte Alkylgruppen mit 1 bis 6 Kohlenstoffatomen, unter dem Begriff "C₁₋₃-Alkyl" dementsprechend verzweigte und unverzweigte Alkylgruppen mit 1 bis 3 Kohlenstoffatomen verstanden. "C₁₋₄-Alkyl" steht entsprechend für verzweigte und unverzweigte Alkylgruppen mit 1 bis 4 Kohlenstoffatomen. Bevorzugt sind Alkylgruppen mit 1 bis 4 Kohlenstoffatomen. Beispielsweise werden hierfür genannt: Methyl, Ethyl, *n*-Propyl, *iso*-Propyl, *n*-Butyl, *iso*-Butyl, *sec-*Butyl, *tert*-Butyl, *n*-Pentyl, *iso*-Pentyl, *neo*-Pentyl oder Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n-*Propyl und *iso*-Propyl, Butyl umfasst *iso*-Butyl, *sec*-Butyl und *tert-*Butyl etc.

Unter dem Begriff "C₁₋₆-Alkylen" (auch soweit sie Bestandteil anderer Reste sind) werden verzweigte und unverzweigte Alkylengruppen mit 1 bis 6 Kohlenstoffatomen verstanden und unter dem Begriff "C₁₋₄-Alkylen" verzweigte und unverzweigte Alkylengruppen mit 1 bis 4 Kohlenstoffatomen verstanden. Bevorzugt sind Alkylengruppen mit 1 bis 4 Kohlenstoffatomen. Beispielsweise werden hierfür genannt: Methylen, Ethylen, Propylen, 1-Methylethylen, Butylen, 1-Methylpropylen, 1,1-Dimethylethylen, 1,2-Dimethylethylen, Pentylen, 1,1-Dimethylpropylen, 2,2, - Dimethylpropylen, 1,2-Dimethylpropylen, 1, 3-Dimethylpropylen oder Hexylen. Sofern nicht anders beschrieben, umfassen die Definitionen Propylen, Butylen, Pentylen und Hexylen alle denkbaren isomeren Formen der jeweiligen Reste gleicher Kohlenstoffanzahl. So umfasst beispielsweise Propyl auch 1-Methylethylen und Butylen umfasst 1-Methylpropylen, 1,1-Dimethylethylen, 1,2-Dimethylethylen.

Sollte die Kohlenstoffkette mit einem Rest substituiert sein, der gemeinsam mit einem oder zwei Kohlenstoffatomen der Alkylenkette einen carbocyclischen Ring mit 3, 5 oder 6 Kohlenstoffatomen bildet, so sind damit unter anderem folgende Beispiele der Ringe umfasst:

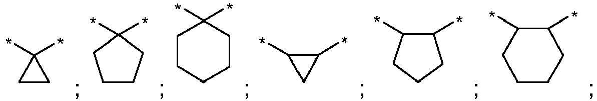

Unter dem Begriff "C₂₋₆-Alkenyl" (auch soweit sie Bestandteil anderer Reste sind) werden verzweigte und unverzweigte Alkenylgruppen mit 2 bis 6 Kohlenstoffatomen und unter dem Begriff "C₂₋₄-Alkenyl" verzweigte und unverzweigte Alkenylgruppen mit 2 bis 4 Kohlenstoffatomen verstanden, soweit sie mindestens eine Doppelbindung aufweisen. Bevorzugt sind Alkenylgruppen mit 2 bis 4 Kohlenstoffatome. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc.

Unter dem Begriff "C₂₋₆-Alkenylen" (auch soweit sie Bestandteil anderer Reste sind) werden verzweigte und unverzweigte Alkenylengruppen mit 2 bis 6 Kohlenstoffatomen verstanden und unter dem Begriff "C₂₋₄-Alkenylen" verzweigte und unverzweigte Alkylengruppen mit 2 bis 4 Kohlenstoffatomen verstanden. Bevorzugt sind Alkenylengruppen mit 2 bis 4 Kohlenstoffatome. Beispielsweise werden hierfür genannt: Ethenylen, Propenylen, 1-Methylethenylen, Butenylen, 1-Methylpropenylen, 1,1-Dimethylethenylen, 1,2-Dimethylethenylen, Pentenylen, 1,1-Dimethylpropenylen, 2,2, -Dimethylpropenylen, 1,2-Dimethylpropenylen, 1,3-Dimethylpropenylen oder Hexenylen. Sofern nicht anders beschrieben, umfassen die Definitionen Propenylen, Butenylen, Pentenylen und Hexenylen alle denkbaren isomeren Formen der jeweiligen Reste gleicher Kohlenstoffanzahl. So umfasst beispielsweise Propenyl auch 1-Methylethenylen und Butenylen umfasst 1-Methylpropenylen, 1,1-Dimethylethenylen, 1,2-Dimethylethenylen.

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindung aufweisen. Bevorzugt sind Alkinylgruppen mit 2 bis 4 Kohlenstoffatome. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc.

Unter dem Begriff "C₂₋₆-Alkinylen" (auch soweit sie Bestandteil anderer Reste sind) werden verzweigte und unverzweigte Alkinylengruppen mit 2 bis 6 Kohlenstoffatomen verstanden und unter dem Begriff "C₂₋₄-Alkinylen" verzweigte und unverzweigte Alkylengruppen mit 2 bis 4 Kohlenstoffatomen verstanden. Bevorzugt sind Alkinylengruppen mit 2 bis 4 Kohlenstoffatome. Beispielsweise werden hierfür genannt: Ethinylen, Propinylen, 1-Methylethinylen, Butinylen, 1-Methylpropinylen, 1, 1-Dimethylethinylen, 1, 2-Dimethylethinylen, Pentinylen, 1, 1-Dimethylpropinylen, 2, 2, -Dimethylpropinylen, 1, 2-Dimethylpropinylen, 1, 3-Dimethylpropinylen oder Hexinylen. Sofern nicht anders beschrieben, umfassen die Definitionen Propinylen, Butinylen, Pentinylen und Hexinylen alle denkbaren isomeren Formen der jeweiligen Reste gleicher Kohlenstoffanzahl. So umfasst beispielsweise Propinyl auch 1-Methylethinylen und Butinylen umfasst 1-Methylpropinylen, 1, 1-Dimethylethinylen, 1, 2-Dimethylethinylen.

Unter dem Begriff "Aryl" (auch soweit sie Bestandteil anderer Reste sind) werden aromatische Ringsysteme mit 6 bis 10 Kohlenstoffatomen verstanden. Beispielsweise werden hierfür genannt: Phenyl oder Naphthyl, bevorzugter Arylrest ist Phenyl. Soweit nicht anders beschrieben, können die Aromaten substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Aryl-C₁₋₆-alkylen" (auch soweit sie Bestandteil anderer Reste sind) werden verzweigte und unverzweigte Alkylengruppen mit 1 bis 6 Kohlenstoffatomen verstanden, die mit einem aromatischen Ringsystem mit 6 oder 10 Kohlenstoffatomen substituiert sind. Beispielsweise werden hierfür genannt: Benzyl, 1- oder 2-Phenylethyl oder 1- oder 2-Naphthylethyl. Soweit nicht anders beschreiben, können die Aromaten substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Heteroaryl-C₁₋₆-alkylen" (auch soweit sie Bestandteil anderer Reste sind) werden - obwohl auch bereits unter "Aryl-C₁₋₆-alkylen umfasst - verzweigte und unverzweigte Alkylengruppen mit 1 bis 6 Kohlenstoffatomen verstanden, die mit einem Heteroaryl substituiert sind.

Ein solches Heteroaryl umfasst fünf- oder sechsgliedrige heterocyclische Aromaten oder 5-10 gliedrige, bicyclische Heteroarylringe die ein, zwei drei oder vier Heteroatome, ausgewählt aus der Gruppe Sauerstoff, Schwefel und Stickstoff enthalten können und so viele konjugierte Doppelbindungen enthalten, dass ein aromatisches Systeme gebildet wird. Als Beispiele für fünf- oder sechsgliedrige heterocyclische Aromaten, werden genannt:

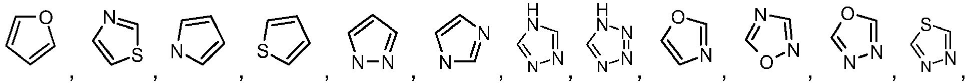

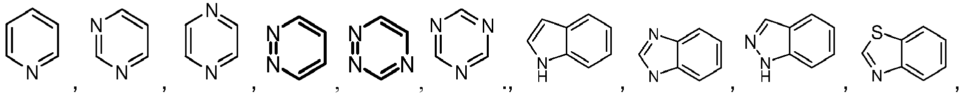

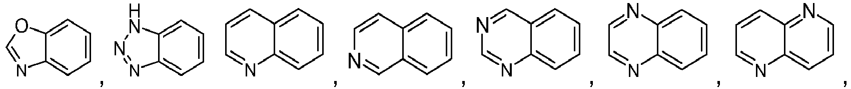

Soweit nicht anders beschrieben, können diese Heteroaryle substituiert sein mit einem oder mehreren Resten ausgewählt aus der Gruppe bestehend aus Methyl, Ethyl, iso-Propyl, tert-Butyl, Hydroxy, Fluor, Chlor, Brom und Jod.

Für die Heteroaryl-C₁-₆-alkylene werden die folgenden Beispiele genannt:

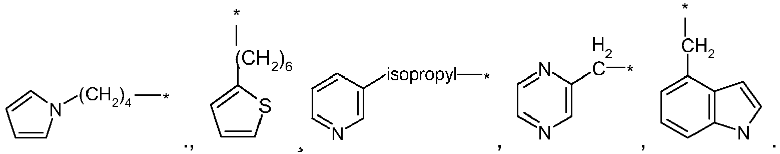

Unter dem Begriff "C₁₋₆-Haloalkyl" (auch soweit sie Bestandteil anderer Reste sind) werden verzweigte und unverzweigte Alkylgruppen mit 1 bis 6 Kohlenstoffatomen verstanden, die mit einem oder mehreren Halogenatomen substituiert sind. Unter dem Begriff "C₁₋₄-Alkyl" verzweigte und unverzweigte Alkylgruppen mit 1 bis 4 Kohlenstoffatomen verstanden, die mit einem oder mehreren Halogenatomen substituiert sind. Bevorzugt sind Alkylgruppen mit 1 bis 4 Kohlenstoffatome. Beispielsweise werden hierfür genannt: CF₃, CHF₂, CH₂F, CH₂CF₃.

Unter dem Begriff "C₃₋₇-cycloalkyl" (auch soweit sie Bestandteil anderer Reste sind) werden cyclische Alkylgruppen mit 3 bis 7 Kohlenstoffatomen verstanden. Beispielsweise werden hierfür genannt: Cyclopropyl, Cyclobutyl, Cyclopentyl, Cyclohexyl oder Cycloheptyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.
Unter dem Begriffe "C₃₋₁₀-Cycloalkyl" werden zudem monocyclische Alkylgruppen mit 3 bis 7 Kohlenstoffatomen und auch bicyclische Alkylgruppen mit 7 bis 10 Kohlenstoffatomen verstanden oder auch monocyclische Alkylgruppen, die durch mindestens eine C₁₋₃Kohlenstoffbrücke überbrückt sind.

Unter dem Begriff "heterocyclische Ringe" oder auch "Heterocyclus" werden -soweit nicht anders beschrieben - fünf-, sechs- oder siebengliedrige, gesättigte, teilweise gesättigte oder ungesättigte heterocyclische Ringe verstanden die ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Obwohl unter dem Begriff "heterocyclische Ringe" oder "Heterocyclus" umfasst, definiert der Begriff "gesättigter Heterocyclus" fünf-, sechs- oder siebengliedrige gesättigte Ringe. Als Beispiele werden genannt:

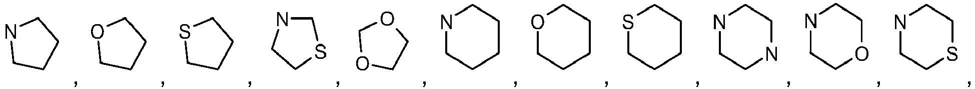

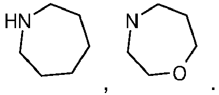

Obwohl unter dem Begriff "heterocyclische Ringe" oder "Heterocyclus" umfasst, definiert der Begriff "teilweise gesättigter Heterocyclus" fünf-, sechs- oder siebengliedrige teilweise gesättigte Ringe, die eine oder zwei Doppelbindungen enthalten, ohne dass so viele konjugierte Doppelbindungen entstehen, dass ein aromatisches System gebildet wird. Als Beispiele werden genannt:

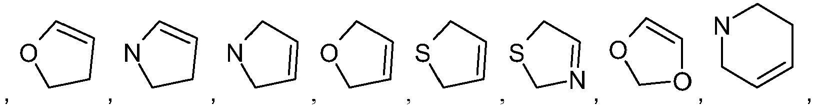

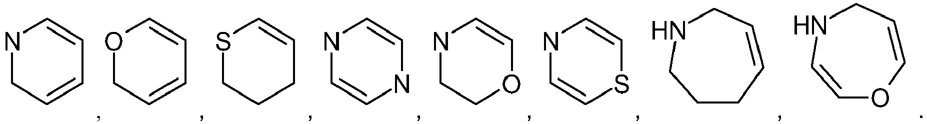

Obwohl unter dem Begriff "heterocyclische Ringe" oder "Heterocyclus" umfasst, definiert der Begriff "heterocyclische, aromatische Ringe" "ungesättigter Heterocyclus" oder "Heteroaryl" fünf- oder sechsgliedrige heterocyclische Aromaten oder 5-10 gliedrige, bicyclische Heteroarylringe die ein, zwei, drei oder vier Heteroatome, ausgewählt aus der Gruppe Sauerstoff, Schwefel und Stickstoff, enthalten können und so viele konjugierte Doppelbindungen enthalten, dass ein aromatisches gebildet Systeme wird. Als Beispiele für fünf- oder sechsgliedrige heterocyclische Aromaten, werden genannt:

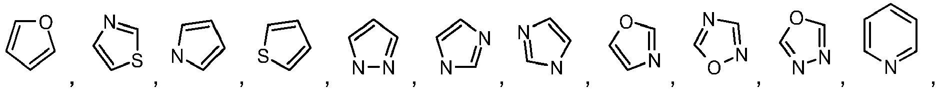

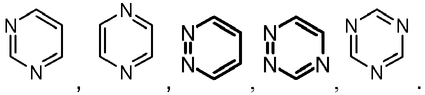

Soweit nicht anders erwähnt, kann ein heterocyclischer Ring (oder "Heterocyclus) mit einer Ketogruppe versehen sein. Als Beispiel hierfür werden genannt.

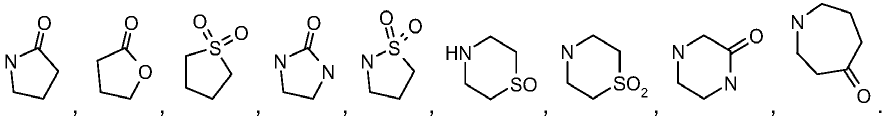

Obwohl unter "Cycloalkyl" bereits umfasst, werden unter dem Unterbegriff "bicyclische Cycloalkyle" in der Regel acht-, neun- oder zehngliedrige, bicyclische Kohlenstoff-Ringe verstanden. Beispielhaft werden genannt:

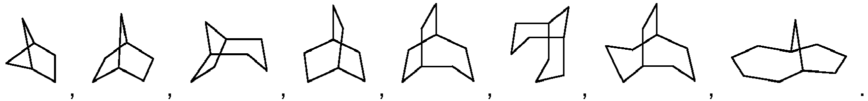

Obwohl unter "Heterocyclus" bereits umfasst, werden unter dem Begriff "bicyclische Heterocyclen" in der Regel acht-, neun- oder zehngliedrige, bicyclische Ringe verstanden, die ein oder mehrere Heteroatome, vorzugsweise 1-4, stärker bevorzugt, 1-3, noch stärker bevorzugt 1-2, insbesondere ein Heteroatom, ausgewählt aus der Gruppe Sauerstoff, Schwefel und Stickstoff enthalten können. Der Ring kann dabei über ein Kohlenstoffatom des Rings oder falls vorhanden über ein Stickstoffatom des Rings mit dem Molekül verknüpft sein. Beispielhaft werden genannt,

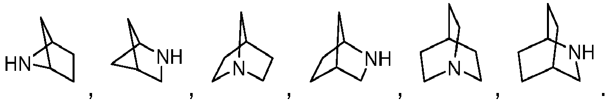

Obwohl unter "Aryl" bereits umfasst, wird unter einem "bicyclischen Aryl" ein 5-10 gliedriger, bicyclischer Arylring verstanden, der so viele konjugierte Doppelbindungen enthält, dass ein aromatisches System gebildet wird. Ein Beispiel für ein bicyclisches Aryl ist Naphthyl.

Obwohl unter "Heteroaryl" bereits umfasst, wird unter einem "bicyclischen Heteroaryl" ein 5-10 gliedriger, bicyclischer Heteroarylring verstanden, der ein, zwei, drei oder vier Heteroatome, ausgewählt aus der Gruppe Sauerstoff, Schwefel und Stickstoff, enthalten kann und so viele konjugierte Doppelbindungen enthält, dass ein aromatisches System gebildet wird.

Obwohl unter dem Begriff "bicyclische Cycloalkyle" oder "bicyclisches Aryl" umfasst, definiert der Begriff "kondensiertes Cycloalkyl" oder "kondensiertes Aryl" bicyclische Ringe, in denen die die Ringe trennende Brücke eine direkte Einfachbindung bedeutet. Als Beispiel für einen kondensiertes, bicyclisches Cycloalkyl werden genannt:

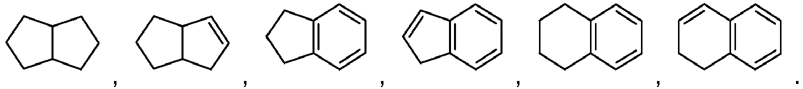

Obwohl unter dem Begriff "bicyclische Heterocyclen" oder "bicyclische Heteroaryle" umfasst, definiert der Begriff "kondensierte, bicyclische Heterocyclen " oder "kondensierte, bicyclische Heteroaryle"bicyclische 5-10 gliedrige Heteroringe die ein, zwei, drei oder vier Heteroatome, ausgewählt aus der Gruppe Sauerstoff, Schwefel und Stickstoff enthalten und in denen die die Ringe trennende Brücke eine direkte Einfachbindung bedeutet. Die "kondensierten, bicyclischen Heteroaryle" enthalten zudem so viele konjugierte Doppelbindungen, dass ein aromatisches System gebildet wird. Beispielhaft werden genannt Pyrrolizin, Indol, Indolizin, Isoindol, Indazol, Purin, Chinolin, Isochinolin, Benzimidazol, Benzofuran, Benzopyran, Benzothiazol, Benzothiazol, Benzoisothiazol, Pyridopyrimidin, Pteridin, Pyrimidopyrimidin,

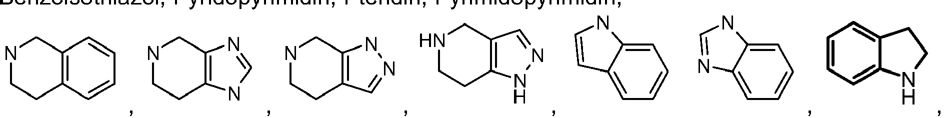

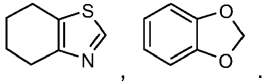

Unter dem Begriff "Spiro-Gruppe" (Spiro) werden 5-10 gliedrige, spirocyclische Ringe verstanden die gegebenenfalls ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Soweit nicht anders erwähnt, kann ein spirocyclischer Ring mit einer Oxo-, Methyl- oder Ethylgruppe versehen sein. Als Beispiele hierfür werden genannt:

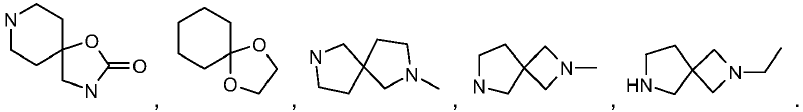

"Halogen" steht im Rahmen der vorliegenden Erfindung für Fluor, Chlor, Brom oder Jod. Sofern nicht gegenteilig angegeben, gelten Fluor, Chlor und Brom als bevorzugte Halogene.

Verbindungen der allgemeinen Formel **1** können Säuregruppen besitzen, hauptsächlich Carboxylgruppen, und/oder basische Gruppen wie z.B. Aminofunktionen. Verbindungen der allgemeinen Formel **1** können deshalb als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Wie vorstehend genannt, können die Verbindungen der Formel **1** in ihre Salze, insbesondere für die pharmazeutische Anwendung, in ihre physiologisch und pharmakologisch verträglichen Salze überführt werden. Diese Salze können einerseits als physiologisch und pharmakologisch verträgliche Säureadditionssalze der Verbindungen der Formel **1** mit anorganischen oder organischen Säuren vorliegen. Andererseits kann die Verbindung der Formel **1** im Falle von R gleich Wasserstoff durch Umsetzung mit anorganischen Basen auch in physiologisch und pharmakologisch verträgliche Salze mit Alkali- oder Erdalkalimetallkationen als Gegenion überführt werden. Zur Darstellung der Säureadditionssalze kommen beispielsweise Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden. Zur Darstellung der Alkali- und Erdalkalimetallsalze der Verbindung der Formel **1** in der R Wasserstoff bedeutet, kommen vorzugsweise die Alkali- und Erdalkalihydroxide und -hydride in Betracht, wobei die Hydroxide und Hydride der Alkalimetalle, besonders des Natriums und Kaliums bevorzugt, Natrium- und Kaliumhydroxid besonders bevorzugt sind.

Gegebenenfalls können die Verbindungen der allgemeinen Formel **1** in ihre Salze, insbesondere für die pharmazeutische Anwendung, in ihre pharmakologisch unbedenklichen Säureadditionssalze mit einer anorganischen oder organischen Säure, überführt werden. Als Säuren kommen hierfür beispielsweise Bernsteinsäure, Bromwasserstoffsäure, Essigsäure, Fumarsäure, Maleinsäure, Methansulfonsäure, Milchsäure, Phosphorsäure, Salzsäure, Schwefelsäure, Weinsäure oder Zitronensäure in Betracht. Ferner können Mischungen der vorgenannten Säuren eingesetzt werden.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

Die erfindungsgemäßen Verbindungen können gegebenenfalls als Racemate vorliegen, sie können aber auch als reine Enantiomere, d.h. in (R)- oder (S)-Form gewonnen werden.

Gegenstand der Erfindung sind die jeweiligen Verbindungen gegebenenfalls in Form der einzelnen optischen Isomeren, Diastereomeren, Mischungen von Diastereomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

Gegenstand der Erfindung sind die jeweiligen Verbindungen der Formel **1** in Form ihrer pharmakologisch verträglichen Salze. Diese pharmakologisch verträglichen Salze der Verbindungen der Formel **1** können ebenfalls in Form ihrer jeweiligen Hydrate (z.B. Monohydrate, Dihydrate etc.) sowie in Form Ihrer jeweiligen Solvate vorliegen.
Unter einem Hydrat der Verbindung nach Formel **1** versteht man im Rahmen der Erfindung ein kristallines, kristallwasserhaltiges Salz der Verbindung nach Formel **1**.

Unter einem Solvat der Verbindung nach Formel **1** versteht man im Rahmen der Erfindung ein kristallines Salz der Verbindung nach Formel **1**, welches Lösungsmittelmoleküle-Moleküle (z.B. Ethanol, Methanol etc) im Kristallgitter enthalten.
Dem Fachmann sind Standardverfahren zur Gewinnung von Hydraten und Solvaten (z.B. das Umkristallisieren aus dem entsprechenden Lösungsmittel bzw. aus Wasser) bekannt.

### 4. HERSTELLUNGSVERFAHREN

Die beanspruchten Verbindungen **1** lassen sich nach bekannten Verfahren (z.B. WO 03/057695) herstellen. Die erfindungsgemässen Beispiele wurden nach Schema 1 dargestellt. 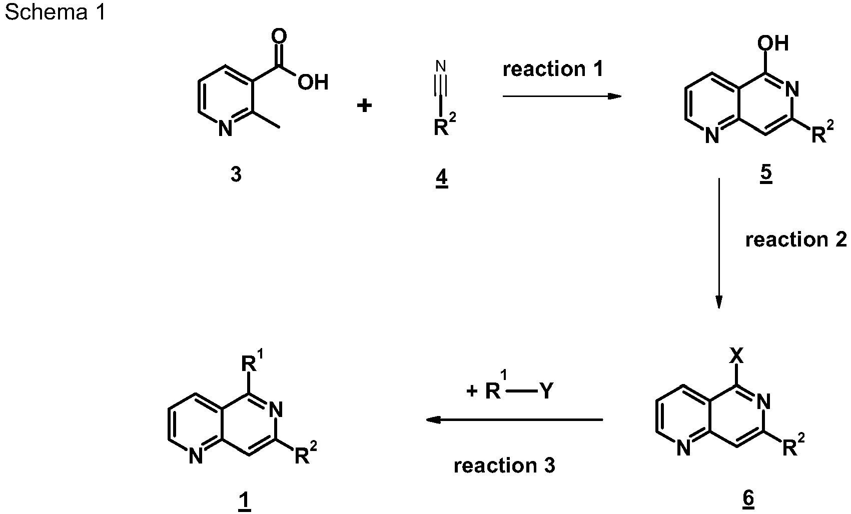
wobei X eine Abgangsgruppe wie z.B. Cl oder Triflat ist,
Y ist -H, -MgBr, -B(OH)₂ ist und
R¹ und R² wie vorstehend definiert sind.

Gegebenenfalls können die Reste R¹ oder R² nachträglich verändert werden z.B durch reduktive Aminierung oder Amidknüpfung.

### 4.1. Zwischenprodukte

### 4.1.1. Synthese der Verbindungen 4 aus Schema 1 (Benzonitril Derivate)

### Synthese von 4-Dimethylamino-3-methyl-benzonitril (4.1)

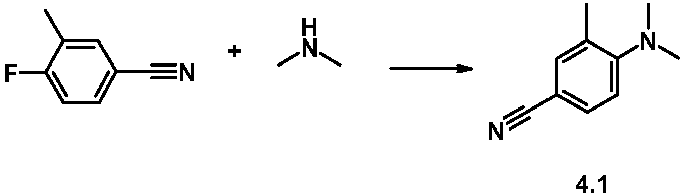

11,0 g (242 mmol) Dimethylamin wurde bis zur Löslichkeitsgrenze in 100 ml Dimethylsulfoxid eingeleitet, anschließend wurden 22,0 g (156 mmol) Kaliumcarbonat und 10,0 g (74 mmol) 4-Fluoro-3-methyl-benzonitril zugegeben. Das Reaktionsgemisch wurde 4 h bei 60°C gerührt, danach wurde es in einen großen Druckkolben überführt und wieder 9,0 g (198 mmol) Dimethylamin eingeleitet. Das Reaktionsgemisch wurde über Nacht bei 85°C gerührt.
Es wurde noch einmal 10,0 g (220 mmol) Dimethylamin eingeleitet und danach 4 h bei 85°C und über Nacht bei 110°C gerührt.
Zum Reaktionsgemisch wurde 500 ml Eiswasser gegeben, anschließend wurde 3 mal mit je 250 ml Ethylacetat extrahiert, die org. Phase wurde getrocknet, filtriert und vom Filtrat das Lösemittel entfernt.
Ausbeute: 12,0 g (75 mmol = 101% d.Th.)
Analytik: HPLC-MS (Methode D): Rₜ: 0,94 min, (M+H)⁺: 161

### 4.1.2. Synthese von R¹-Derivaten (Amin-Derivate)

### Synthese von 4-(2-Aminoethyl)-3-hydroxy-1H-pyrazol (für Beispiel 8)

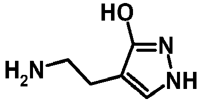

4-(2-Aminoethyl)-3-hydroxy-1H-pyrazol wurde nach folgender Vorschrift hergestellt: Kralj, David; Groselj, Uros; Meden, Anton; Dahmann, Georg; Stanovnik, Branko; Svete, Jurij. A simple synthesis of 4-(2-aminoethyl)-5-hydroxy-1H-pyrazoles. Tetrahedron (2007), 63 (45), 11213-11222.

### Synthese von 4-(2-Hydroxy-ethyl)-pyrazol-1-carbonsäure tert-butyl ester (für Beispiel 12)

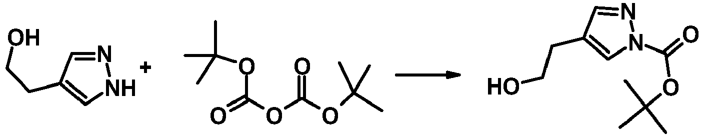

1,10 g (9,81 mmol) 2-(1*H*-Pyrazol-4-yl)-ethanol, 0,20 g (1,64 mmol) 4-Dimethylaminopyridin und 1,50 ml (10,3 mmol) Triethylamin wurden in 30 ml Dichlormethan vorgelegt, das Reaktionsgemisch wurde auf 0°C abgekühlt und 2,20 g (9,88 mmol) Kohlensäuretertbutylester wurde langsam zugegeben. Der Ansatz wurde 2 h bei Raumtemperatur gerührt und weiter über Nacht bei Raumtemperatur. Es wurde mit 50 ml Dichlormethan verdünnt und mit 10%iger Zitronensäure Lösung und 15%iger Kaliumcarbonat Lösung gewaschen, die organische Phase wurde getrocknet, filtriert und vom Filtrat das Lösemittel entfernt.
Ausbeute: 2,00 g (9,42 mmol = 96% d.Th.)

### 4.2. Reaktion 1 des Schemas 1: Synthese von Verbindungen der Formel 5

### Synthese von 7-(4-Dimethylamino-3-methylphenyl)-[1,6]naphthyridin-5-ol (5.1)

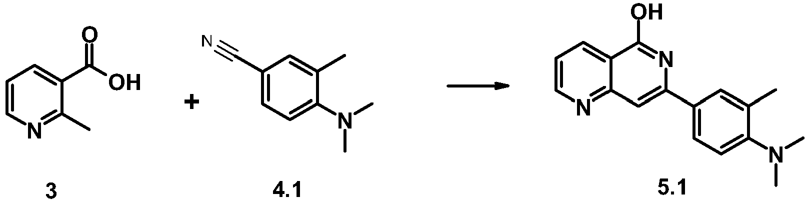

Die Reaktion wurde unter Argonatmosphäre ausgeführt.
4,40 g (31 mmol) 2-Methyl-nicotinsäure wurde in 75 ml Tetrahydrofuran suspendiert, und auf80°C abgekühlt. 49 ml (98 mmol) Lithiumdiisopropylamid (2,0 mol/l in Tetrahydrofuran) wurde innerhalb von 30 Minuten zugetropft und 2 h bei -60°C gerührt.
Danach wurde wieder bei -60°C eine Lösung von 5,0 g (31 mmol) 4-Dimethylamino-3-methylbenzonitril in Tetrahydrofuran innerhalb von 30 Minuten zugetropft. Danach wurde das Reaktionsgemisch für 4 h bei 0°C gerührt.
Die Suspension wurde mit 100 ml Wasser versetzt und das Lösemittel abdestilliert. Der wässrige Rückstand wurde mit 20 ml Ethylacetat versetzt und 20 min gerührt, anschließend wurde der Niederschlag abgesaugt und getrocknet.
Ausbeute: 3,0 g (10,70 mmol = 34% d. Th.)
Analytik: HPLC (Methode D): Rₜ = 0.96 min. ESI-MS: (M+H)⁺: 280

### 4.3. Reaktion 2 des Schemas 1: Synthese von Verbindungen der Formel 6

### 4.3.1 Synthese von 5-Chloro-7-(4-dimethylamino-3-methylphenyl)-[1,6]naphthyridin (6.1)

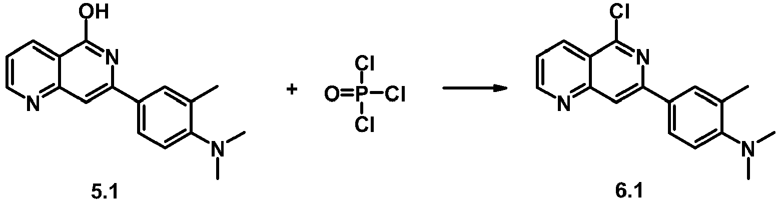

3,0 g (10,7 mmol) **5.1** und 0,50 ml (2,3 mmol) *N,N*-Diethylanilin wurden in 25 ml (272 mmol) Phosphoroxychlorid für 1 h bei 115°C gerührt.
Das Reaktionsgemisch wurde eingeengt, der Rückstand wurde mit ca. 50 ml Wasser versetzt, mit NaHCO₃-Lösung neutral gestellt und mit Dichlormethan extrahiert. Die organische Phase wurde mit MgSO4 getrocknet und eingeengt.
Ausbeute: 1,90 g Öl (4,7 mmol = 44% d. Th.)
Analytik: HPLC (Methode D): Rₜ = 1,25 min. ESI-MS: (M+H)⁺: 298/300 (Cl)

### 4.3.2. Synthese von 7-(4-Dimethylamino-3-methylphenyl)-[1,6]naphthyridin-5-yl-trifluormethansulfonsäureester (6.2)

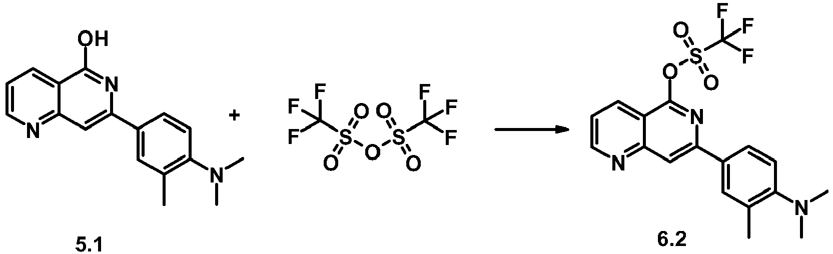

2,2 mL (12 mmol) Trifluormethansulfonsäureanhydrid wurde in 25 mL Dichlormethan vorgelegt und unter Eiskühlung 3,40 g (12 mmol) **5.1**, gelöst in 1,1 ml (13 mmol) Pyridin und 25 mL Dichlormethan, zugetropft. Das Reaktionsgemisch wurde nach der Zugabe auf Raumtemperatur erwärmt und über Nacht bei 25°C gerührt.
Anschließend wurde nochmal 1,5 ml Trifluormethansulfonsäureanhydrid und 3 mL Pyridin zugegeben. Nach einer Stunde wurde mit 100 mL Eiswasser verdünnt, die organische Phase mit NaCl-Lösung gewaschen, mit MgSO₄ getrocknet und eingeengt. Der Rückstand wurde noch 2x in Toluol aufgenommen und anschliessend eingeengt.
Ausbeute: 4,70 g (8,4 mmol = 69% d.Th.)
Analytik: HPLC (Methode D): Rₜ = 1,40 min ESI-MS (M+H)⁺ = 412

### 4.4. Reaktion 3 des Schemas 1 (Synthese der Patentbeispiele der Formel 1)

### Beispiele 1+ 2:

### cis und trans 5-(4-Hydroxycyclohexyl)-7-(4-dimethylamino-3-methylphenyl)-[1,6]naphthyridin

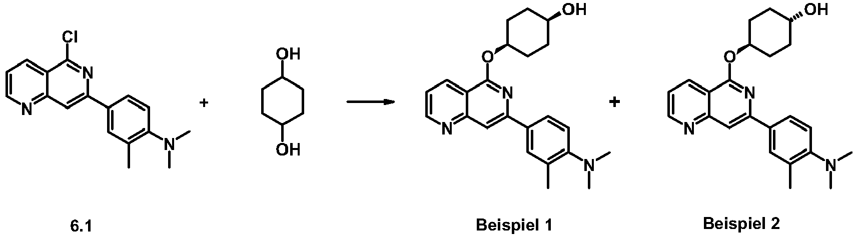

200 mg (1,7 mmol) Cyclohexandiol wurde in 1,5 ml Dimethylacetamid vorgelegt und 47 mg (1,17 mmol) Natriumhydrid (60%) zugegeben und 15 min bei Raumtemperatur gerührt. Anschließend wurde 100 mg (0,31 mmol) **6.1** zugegeben und 2 h bei 70°C gerührt. Das Reaktionsgemisch wurde mit Dichlormethan verdünnt und 1x mit Wasser extrahiert. Die organische Phase wurde mit MgSO₄ getrocknet und eingeengt. Der Rückstand wurde chromatographisch gereinigt (RP-HPLC-MS). Die entsprechenden Fraktionen wurden gefriergetrocknet.
Ausbeute Beispiel 1: 9 mg (0,018 mmol = 5,5% d.Th.)
Analytik: HPLC-MS (Methode D): Rₜ: 1,13 min, (M+H)⁺: 378
Ausbeute Beispiel 2: 42 mg (0,085 mmol = 25% d.Th.)
Analytik: HPLC-MS (Methode D): Rₜ: 1,15 min, (M+H)⁺: 378

Analog der Beispiele 1+2 wurde Beispiel 7 hergestellt.

### Beispiel 3:

### 5-(Aminomethylpyrrolidin-1-yl)-7-(4-dimethylamino-3-methylphenyl)-[1,6]naphthyridin

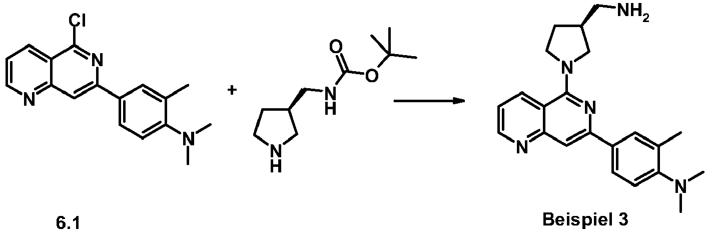

100 mg (0,37 mmol) **6.1** wurde mit 210 mg (1,04 mmol) (*R*)-3-*N*-*tert*-Butyloxycarbonyl-aminomethylpyrrolidin für 60 min bei 100°C gerührt. Zum Ansatz wurde 1 ml Trifluoressigsäure und Acetonitril gegeben und für 30 min bei 50°C gerührt. Es wurde mit Acetronitril/Wasser verdünnt und chromatographisch gereinigt (RP-HPLC-MS). Die entsprechenden Fraktionen wurden gefriergetrocknet.
Ausbeute: 120 mg (0,33 mmol = 99% d.Th.)
Analytik: HPLC-MS (Methode D): Rₜ: 1,00 min, (M+H)⁺: 362

### Beispiel 4

### 5-(Pyrrolidin-3-yl-methoxy)-7-(4-dimethylamino-3-methylphenyl)-[1,6]naphthyridin

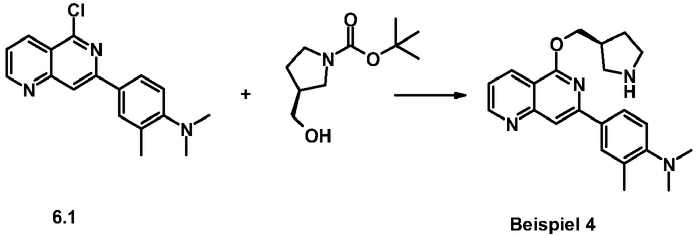

118 mg (0,59 mmol) (S)-3-Hydroxymethylpyrrolidincarbonsäure *tert* butylester und 23,5 mg (0,59 mmol) Natriumhydrid (60%) wurden in 0,5 ml Dimethylacetamid vorgelegt und 15 min bei Raumtemperatur gerührt. Anschließend wurde 70 mg (0,24 mmol) **6.1** zugegeben und 2 h bei 70°C gerührt.
Es wurde 1 mL Trifluoressigsäure zugegeben und über 2 Tage bei 25°C gerührt. Das Reaktionsgemisch wurde chromatographisch gereinigt (RP-HPLC-MS). Die entsprechenden Fraktionen wurden gefriergetrocknet.
Ausbeute: 110 mg (0,23 mmol = 98% d.Th.)
Analytik: HPLC-MS (Methode D): Rₜ: 1,05 min, (M+H)⁺: 363

Analog zu Beispiel 4 wurden die folgenden Verbindungen erhalten: Beispiel 14, 15, 18, 19.

### Beispiel 6:

### 5-(3-Pyrazolyl-ethylamino)-7-(4-dimethylamino-3-methylphenyl)-[1,6]naphthyridin

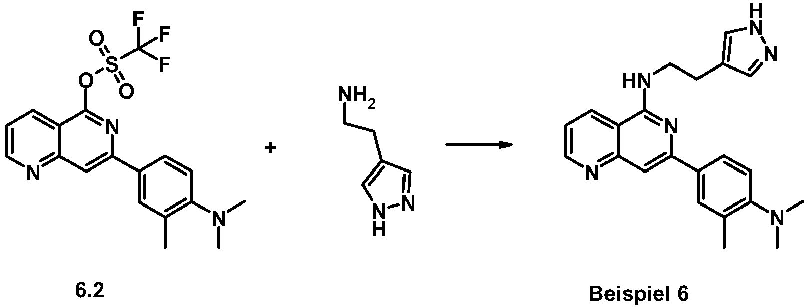

100 mg (0,18 mmol) **6.2,** 65 mg (0,35 mmol) 2-(1*H*-Pyrazol-4-yl)ethylamin dihydrochlorid und 120 µl (0,71 mmol) Diisopropylethylamin wurden in 0,5 ml Dimethylacetamid 2 h bei 80°C gerührt. Der Ansatz wurde chromatographisch gereinigt (RP-HPLC-MS). Die entsprechenden Fraktionen wurden gefriergetrocknet.
Ausbeute: 70,00 mg (0,14 mmol = 81% d.Th.)
Analytik: HPLC-MS (Methode D): Rₜ: 1,05 min, (M+H)⁺: 373

Analog Beispiel 6 wurden die Beispiele 8-11, 13, 22, 24-30 erhalten.

### Beispiel 12:

### 5-(4-Hydroxyethyl-pyrazolyl)-7-(4-dimetyhlamino-3-methylphenyl)-[1,6]naphthyridin

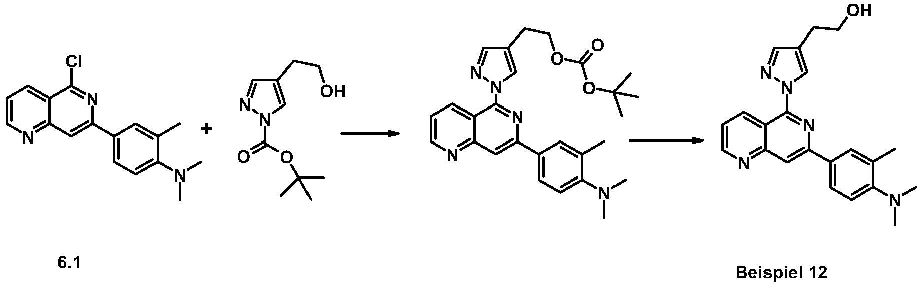

110 mg (0,49 mmol) 4-(2-Hydroxy-ethyl)-pyrazol-1-carbonsäure *tert*-butylester wurde in 0,5 mL Dimethylacetamid gelöst, 22 mg 0,5 mmol) NaH (55%) zugegeben und 15 min gerührt. Dann wurde 100 mg (0,34 mmol) **6.1** zugegeben und für 1 h bei 50°C gerührt. Der Ansatz wurde mit Acetonitril, Wasser und Trifluoressigsäure verdünnt und chromatographisch gereinigt (RP-HPLC).
Ausbeute: 50 mg (0,11 mmol = 31% d. Th).
Analytik: HPLC-MS (Methode D): Rₜ: 1,45 min, (M+H)⁺: 474 50 mg (0,11 mmol) *tert*-Butyl-2-{1-[7-(4-dimethylamino-3-methyl-phenyl)-[1,6]naphthyridin-5-yl]-1H-pyrazol-4-yl}-kohlensäureester wurde in 2 ml (8 mmol) dioxanische Salzsäure (4mol/l) gelöst und 2 h bei 50°C gerührt. Das Reaktionsgemisch wurde eingeengt, der Rückstand wurde chromatographisch gereinigt (RP-HPLC-MS), die entsprechenden Fraktionen wurden gefriergetrocknet.
Ausbeute: 30 mg (0,08 mmol = 76% d.Th.)
Analytik: HPLC-MS (Methode D): Rₜ: 1,17 min, (M+H)⁺: 374

### Beispiel 17:

### 5-Methoxy-7-(4-dimethylamino-3-methylphenyl)-[1,6]naphthyridin

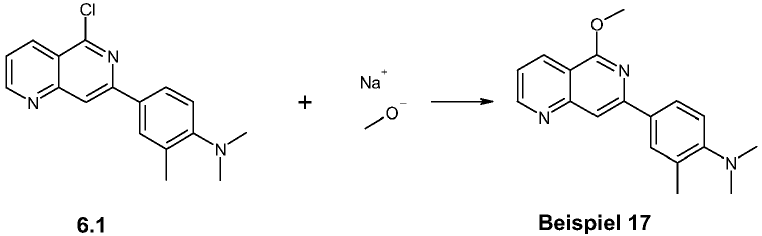

100 mg (0,31 mmol) **6.1** wurde in 0,5 ml Dimethylacetamid vorgelegt und anschließend 60 mg (1,1 mmol) Natriummethanolat zugegeben. Das Reaktionsgemisch wurde 1 h bei 50°C gerührt. Der Ansatz wurde chromatographisch gereinigt (RP-HPLC-MS), die entsprechenden Fraktionen wurden gefriergetrocknet.
Ausbeute: 120 mg (0,29 mmol = 88% d.Th.)
Analytik: HPLC-MS (Methode D): Rₜ: 1,14 min, (M+H)⁺: 294

### Beispiel 20:

### 5-(Cyclopropylamino)-7-(4-dimethylamino-3-methylphenyl)-[1,6]naphthyridin

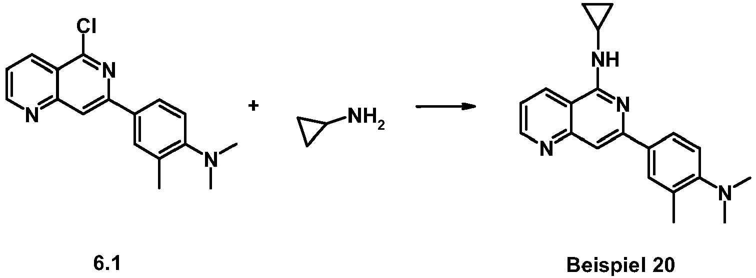

100 mg (0,34 mmol) **6.1,** 75 µL (1,07 mmol) Cyclopropylamin wurden in 500 µl Dimethylformamid gelöst und über Nacht bei 100°C gerührt. Der Ansatz wurde mit Acetonitril/Wasser und Trifluoressigsäure versetzt und chromatographisch gereinigt (RP-HPLC). Die entsprechenden Fraktionen wurden gefriergetrocknet.
Ausbeute: 40 mg (0,131 mmol = 40% d.Th.)
Analytik: HPLC-MS (Methode D): Rₜ: 1,07 min, (M+H)⁺: 319

Analog Beispiel 20 wurden die Beispiele 5, 16 erhalten.

### Beispiel 21:

### 5-(Pyrrolidin-2-on-4-yl-methylhydroxy)-7-(4-dimethylamino-3-methylphenyl)-[1,6]naphthyridin

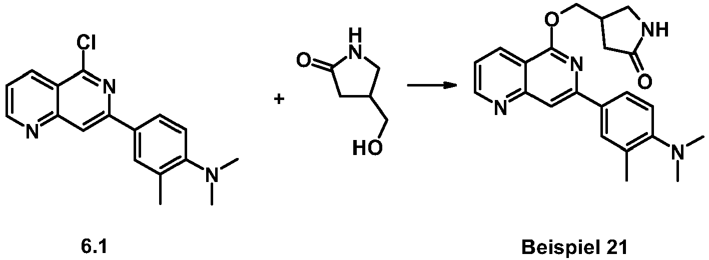

45 mg (0,38 mmol) 4-Hydroxymethylpyrrolidin-2-on wurde in 1 ml Dimethylacetamid vorgelegt und 16 mg (0,29 mmol) Natriumhydrid (60%) zugegeben und 15 min bei Raumtemperatur gerührt. Anschließend wurde 100 mg (0,34 mmol) **6.1** zugegeben und 2 h bei 50°C und über Nacht bei 25°C gerührt. Das Reaktionsgemisch wurde chromatographisch gereinigt (RP-HPLC-MS). Die entsprechenden Fraktionen wurden gefriergetrocknet.
Ausbeute: 120 mg (0,32 mmol = 95% d.Th.)
Analytik: HPLC-MS (Methode D): Rₜ: 1,09 min, (M+H)⁺: 377

Analog Beispiel 21 wurde Beispiel 23 erhalten.

### 4.5 Chromatographische Methoden (HPLC-MS Methoden)

Die nach den obigen Synthese-Schema hergestellten Beispielverbindungen wurden durch folgende chromatographische Methode, die - sofern sie durchgeführt wurden - im einzelnen in der Tabelle 1 angegeben sind, charakterisiert.

### Methode D

Waters ZMD, Alliance 2690/2695 HPLC, Waters 996/2996 Diodenarraydetektor

Als mobile Phase wurde eingesetzt:
A: Wasser mit 0.10% TFA
B: Acetonitril mit 0.10% TFA

| Zeit in min | %A | %B | Flussrate in ml/min |
|---|---|---|---|
| 0.00 | 95 | 5 | 2.80 |
| 0.30 | 95 | 5 | 2.80 |
| 1.60 | 2 | 98 | 2.80 |
| 1.90 | 2 | 98 | 2.80 |
| 2.00 | 95 | 5 | 2.50 |

Als stationäre Phase dient eine Säule Merck Chromolith^{™} Flash RP-18e, 3 mm x 100 mm (Säulentemperatur: konstant bei 25°C).

Die Diodenarraydetektion erfolgte im Wellenlängenbereich 210-400 nm.

### 5. BEISPIELE

Die folgenden Beispiele wurden analog den oben gezeigten Synthesevorschriften herstellt (je nach Kennzeichnung in der Tabelle 1). Diese Verbindungen sind als SYK-Inhibitoren geeignet und besitzen IC₅₀-Werte kleiner oder gleich 1 µmol. Die Inhibitionen (in %) bei 1 µM der einzelnen Beispiel-Substanzen sind in der nachfolgenden Beispiel-Tabelle eingefügt und wurden wie folgt bestimmt:

### Syk Kinase Test

Rekombinantes humanes Syk wurde als Fusionsprotein mit einem N-terminalen GST-tag exprimiert, Affinitäts-gereinigt und bei einer Konzentration von ca. 50 - 100 µM im Testpuffer (25 mM HEPES pH7,5; 25 mM MgCl₂;5 mM MnCl₂; 50 mM KCl; 0,2% BSA; 0,01% CHAPS; 100 µM Na₃VO₄; 0,5 mM DTT) und 10% Glyzerin bei -80°C bis zur Verwendung tiefgefroren.
Die katalytische Aktivität des GST-Syk Kinase Fusionsproteins wurde mittels des Kinase Glo^{®} Lumineszenz Kinasetests der Firma Promega bestimmt. In diesem homogenen Test wird die nach der durchgeführten Kinasereaktion verbleibende ATP Menge über eine Luciferin-Luciferase Reaktion mittels Lumineszenz quantifiziert. Das erhaltene Lumineszenzsignal korreliert mit der noch vorhandenen ATP Menge und korreliert somit invers mit der Aktivität der Proteinkinase.

### Durchführung

Die Testsubstanzen wurden in 100 % DMSO bei einer Konzentration von 10 mM gelöst und in DMSO auf eine Konzentration von 1 mM verdünnt. Alle weiteren Verdünnungen der Substanzen wurden mit 7.5 % DMSO in Testpuffer durchgeführt, bis eine Konzentration erreicht wurde, die 7.5-fach über der finalen Testkonzentration lag (finale Konzentration der Substanzen: im Normalfall 30 µM bis 1 nM). Je 2 µl dieser Verdünnungen wurden in eine 384-Loch Optiplate (Perkin Elmer, # 6007290) transferiert. GST-Syk wurde auf 6.0 nM im Testpuffer verdünnt und 10 µl dieser Verdünnung wurden in den Kinasetest eingesetzt (finale Konzentration von Syk = 4 nM in einem Gesamtvolumen 15 µl). Nach einer 15-minütigen Inkubation bei Raumtemperatur wurden pro Loch 3 µl einer Mischung von 750 nM ATP und 100 µg/ml poly (L-Glutamicacid L-Tyrosine 4:1), Fluka # 81357) in Testpuffer zugesetzt und anschließend weitere 60 Minuten bei Raumtemperatur inkubiert.
Positive Kontrollen sind die Reaktionsgemische, die keine Testsubstanz enthalten; negative Kontrollen sind Reaktionsgemische, die keine Kinase enthalten.
Nach 60 Minuten werden pro Loch 10 µl Kinase-Glo^{®} Lösung (Promega, Cat. # V6712) (auf Raumtemperatur erwärmt) hinzugefügt und für weitere 15 Minuten bei Raumtemperatur inkubiert. Anschließend wurden die Platten werden in einem Microplate Scintillation und Lumineszenzzähler ausgelesen (PerkinElmer / Wallac: MicroBeta TRILUX 1450 LSC & Luminescence Counter).

### Datenauswertung und Berechnung:

Die Ausgabedatei des "MicroBeta TRILUX " ist eine Textdatei, welche die Lochnummer und die Messwerte enthält. Zur Auswertung wurde der Messwert der Negativkontrolle als 100 % Inhibition und der Messwert der Positivkontrolle als 0% Inhibition festgesetzt. Anschließend wurde daraus für den Messwert einer jeden Substanzkonzentration der % Inh. Wert mittels eines "MS-Excel - VB - Makros" errechnet. Im Normalfall liegen die errechneten % Inhibitionswerte zwischen 100% und 0 % Inhibition, jedoch können in Einzelfällen auch Werte außerhalb dieser Grenzen auftreten. Die IC₅₀ Werte wurden aus den % Inhibitions-Werten mit der Software "GraphPadPrism" (Version 5) (GraphPad Software Inc.) berechnet.

### Tabelle 1: Beispiele der Formel 1

| Die folgenden Beispiele der Formel 1 | | | | | |
|---|---|---|---|---|---|
| 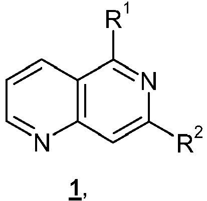 | | | | | |

| mit den folgenden Eigenschaften wurden nach den zuvor genannten Synthesevorschriften hergestellt, wobei X₁ die Stelle bezeichnet, an der der Rest R¹ mit der Struktur der Formel 1 verknüpft ist, und wobei X₂ die Stelle bezeichnet, an der der Rest R² mit der Struktur der Formel 1 verknüpft ist: | | | | | |
|---|---|---|---|---|---|
| Bsp | R¹ | R² | Retentionszeiten | SYK Inh % bei 1 µM | Herstellverfahren |
| **1** | 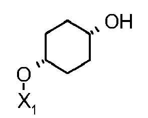 | 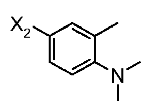 | 1,13 | 85,2 | Siehe Beschreibung |
| **2** | 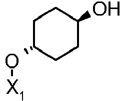 | 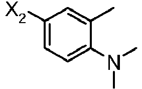 | 1,15 | 71,9 | Siehe Beschreibung |
| **3** | 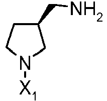 | 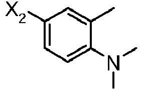 | 0,99 | 88,2 | Siehe Beschreibung |
| **4** | 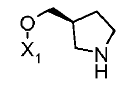 | 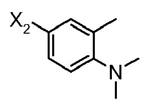 | 1,05 | 87,5 | Siehe Beschreibung |
| **5** | 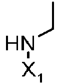 | 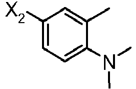 | 1,07 | 97,8 | Analog Beispiel 20 |
| **6** | 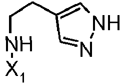 | 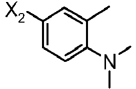 | 1,05 | 85,2 | Siehe Beschreibung |
| **7** | 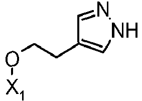 | 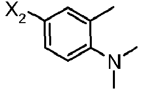 | 1,13 | 71,9 | Analog Beispiel 1 |
| **8** | 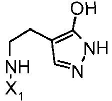 | 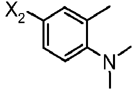 | 1,06 | 88,2 | Analog Beispiel 6 |
| **9** | 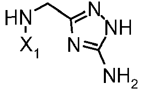 | 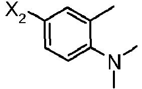 | 1,01 | 87,5 | Analog Beispiel 6 |
| **10** | 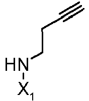 | 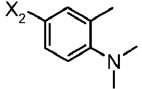 | 1,11 | 102 | Analog Beispiel 6 |
| **11** | 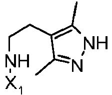 | 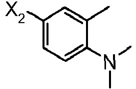 | 1,06 | 97,8 | Analog Beispiel 6 |
| **12** | 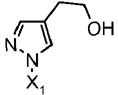 | 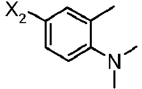 | 1,17 | 105 | Siehe Beschreibung |
| **13** | 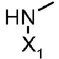 | 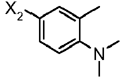 | 1,04 | 90,5 | Analog Beispiel 6 |
| **14** | 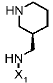 | 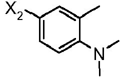 | 1,02 | 90,6 | Analog Beispiel 4 |
| **15** | 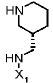 | 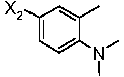 | 1,02 | 73,4 | Analog Beispiel 4 |
| **16** | 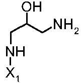 | 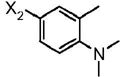 | 0,99 | 81,4 | Analog Beispiel 20 |
| **17** | 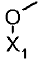 | 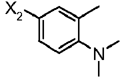 | 1,14 | 73,2 | Siehe Beschreibung |
| **18** | 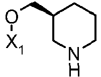 | 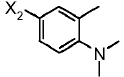 | 1,06 | 68 | Analog Beispiel 4 |
| **19** | 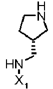 | 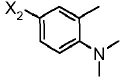 | 1,01 | 85,8 | Analog Beispiel 4 |
| **20** | 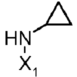 | 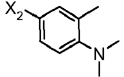 | 1,07 | 90,2 | Siehe Beschreibung |
| **21** | 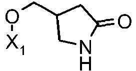 | 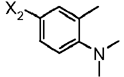 | 1,09 | 92,9 | Siehe Beschreibung |
| **22** | 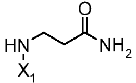 | 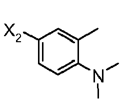 | 1,00 | 90,1 | Analog Beispiel 6 |
| **23** | 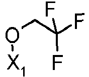 | 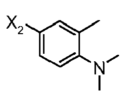 | 1,34 | 80,5 | Analog Beispiel 21 |
| **24** | 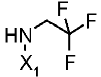 | 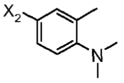 | 1,14 | 93,3 | Analog Beispiel 6 |
| **25** | 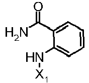 | 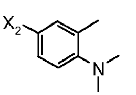 | 1,17 | 97,6 | Analog Beispiel 6 |
| **26** | 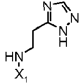 | 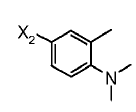 | 1,02 | 87,5 | Analog Beispiel 6 |
| **27** | 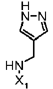 | 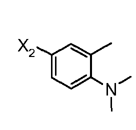 | 1,05 | 88,7 | Analog Beispiel 6 |
| **28** | 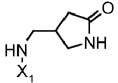 | 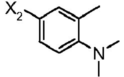 | 1,02 | 95,9 | Analog Beispiel 6 |
| **29** | 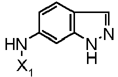 | 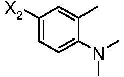 | 1,14 | 63 | Analog Beispiel 6 |
| **30** | 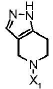 | 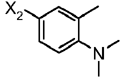 | 1,07 | 80,6 | Analog Beispiel 6 |

### 6. INDIKATIONEN

Wie gefunden wurde, zeichnen sich die Verbindungen der Formel **1** durch vielfältige Anwendungsmöglichkeiten auf therapeutischem Gebiet aus. Hervorzuheben sind solche Anwendungsmöglichkeiten, für welche die erfindungsgemäßen Verbindungen der Formel **1** aufgrund ihrer pharmazeutischen Wirksamkeit als SYK-Inhibitor bevorzugt zur Anwendung gelangen können. Beispielhaft genannt seien Atemwegserkrankungen, allergische Erkrankungen, Osteoporose, gastrointestinale Erkrankungen oder Beschwerden, Immun- oder Autoimmunerkrankungen, allergische Erkrankungen, entzündliche Erkrankungen, z.B. entzündliche Erkrankungen der Gelenke, der Haut und der Augen sowie Erkrankungen des periphären oder zentralen Nervensystems.

Hierbei bevorzugt genannt sei die Vorbeugung und Behandlung von Atemwegs- oder Lungenerkrankungen, welche mit einer erhöhten Schleimproduktion, Entzündungen und/oder obstruktiven Erkrankungen der Atemwege einher gehen. Beispielhaft hierfür seinen genannt Asthma, pediatrisches Asthma, ARDS (Adultes respiratorisches Distress Syndrom), akute, allergische oder chronische Bronchitis, chronisch obstruktive Bronchitis (COPD) (inklusive der Behandlung Rhinovirus-induzierter Exazerbationen), Husten, allergische Rhinitis oder Sinusitis, allergische Rhinokonjunktivitis, chronische Rhinitis oder Sinusitis, Alveolitis, Farmers' Krankheit, hyperreaktive Atemwege, infektiöse Bronchitis oder Pneumonitis, Bronchiectasien, Lungenfibrose, Bronchialödem, Lungenödem, Pneumonie oder interstitielle Pneumonie ausgelöst durch verschiedene Ursachen wie Aspiration, Inhalation von toxischen Gasen oder Bronchitis, Pneumonie oder interstitielle Pneumonie ausgelöst durch Herzinsuffizienz, Bestrahlung, Chemotherapie zystische Fibrose oder Mukoviszidose, alpha1-Antitrypsin-Mangel.

Die erfindungsgemäßen Verbindungen eignen sich ebenfalls bevorzugt zur Behandlung von allergischen Erkrankungen wie beispielsweise Allergische Rhinitis, allergische Rhinokonjunktivitis, Allergische Konjunktivitis, allergische Dermatitis und Kontakt Dermatitis, Urtikaria / Angioödema und allergische Dermatitis.

Ebenfalls bevorzugt genannt sei die Behandlung von entzündlichen Erkrankungen des Gastrointestinaltraktes. Beispielhaft hierfür seinen genannt Morbus Crohn und Colitis ulcerosa.

Die erfindungsgemäßen Verbindungen eignen sich ebenfalls bevorzugt zur Behandlung von entzündlichen Erkrankungen der Gelenke oder entzündliche Erkrankungen der Haut und der Augen. Beispielhaft seien hierfür genannt rheumatoide Arthritis, Antikörper-basierende Glomerulonephritis, Psiorasis, Kawasaki Syndrom,Zöliakie (Sprue) und Wegener-Granulomatose.

Die erfindungsgemäßen Verbindungen eignen sich ebenfalls bevorzugt zur Behandlung von Autoimmunerkrankungen. Beispielhaft seien hierfür genannt Hepatitis (autoimunn-basierend), Lupus erythematodes, Anti-Phospholipid Syndrom, Berger-Krankheit, Evans Syndrom, Immunhämolytische Anämie, ITP (idiopathische thrombozytopenische Purpura; adult, neonatal und peadriatisch), Myastenia Gravis, Sjögren Syndrome und Sklerodermie.

Die erfindungsgemäßen Verbindungen eignen sich ebenfalls bevorzugt zur Behandlung von B-Zell Lymphomen.

Des Weiteren bevorzugt genannt sei die Vorbeugung und Behandlung von Erkrankungen des periphären oder zentralen Nervensystems. Beispielhaft hierfür seien genannt akute und chronische Multiple Sklerose oder nicht familiäre Lateral Sklerose.

Des Weiteren bevorzugt genannt sei die Vorbeugung und Behandlung von osteoporotischen Erkrankungen wie beispielsweise Krankheits-assoziierte Osteopenie, Osteoporose und Osteolytische Erkrankungen.

Besonders bevorzugt betrifft die vorliegende Erfindung die Verwendung von Verbindungen der Formel **1** zur Herstellung eines Arzneimittels zur Behandlung der Erkrankungen ausgewählt aus der Gruppe bestehend aus Asthma, COPD, allergische Rhinitis, Adultes respiratorisches Distress Syndrom, Bronchitis, allergische Dermatitis, Kontakt Dermatitis, ITP, rheumatoide Arthritis und allergische Rhinokonjunktivitis.

Am meisten bevorzugt ist die Verwendung der Verbindungen der Formel **1** zur Behandlung von einer Erkrankung ausgewählt aus der Gruppe bestehend aus Asthma, allergische Rhinitis, rheumatoide Arthritis, allergische Dermatitis und COPD.

### 7. KOMBINATIONEN

Die Verbindungen der Formel **1** können allein oder in Kombination mit anderen erfindungsgemäßen Wirkstoffen der Formel **1** zur Anwendung gelangen. Gegebenenfalls können die Verbindungen der Formel **1** auch in Kombination mit weiteren pharmakologisch aktiven Wirkstoffen eingesetzt werden. Bevorzugt gelangen hierbei solche Wirkstoffe zur Anwendung, die beispielsweise ausgewählt sind aus der Gruppe bestehend aus Betamimetika, Anticholinergika, Corticosteroiden, PDE4-Inhibitoren, LTD4-Antagonisten, EGFR-Hemmern, MRP4-Inhibitoren, Dopamin-Agonisten, H1-Antihistaminika, PAF-Antagonisten, iNos-Inhibitoren, PI3-Kinase-Inhibitoren, CCR3-Antagonisten, CCR2-Antagonisten, CCR1-Antagonisten, IKK2-Inhibitoren, A2a-Agonisten, alpha-4-Integrin-Inhibitoren, CRTH2-Antagonisten, Histamin 1, kombinierte H1/H3-Antagonisten, p38 Kinase Inhibitoren, Methylxanthine, ENaC-Inhibitoren, CXCR1-Antagonisten, CXCR2-Antagonisten, ICE-Inhibitoren, LTB4-Antagonisten, 5-LO Antagonisten, FLAP-Antagonisten. LTB4-Antagonisten; Cromoglicin, dissoziierte-Glucocorticoidmimetika, anti-TNF-Antikörper, anti-GM-CSF Antikörper, anti-CD46-Antikörper, anti-IL-1-Antikörper, anti-IL-2-Antikörper, anti-IL-4-Antikörper, anti-IL-5-Antikörper, anti-IL-13-Antikörper, anti-IL-4/IL-13-Antikörper, oder zwei- oder dreifach Kombinationen davon, wie beispielsweise Kombinationen von Verbindungen der Formel **1** mit ein oder zwei Verbindungen aus der Gruppe bestehend aus
- Betamimetika, Corticosteroiden, SYK-Inhibitoren der Formel **1**, EGFR-Hemmer und PDE4-Antagonisten,
- Anticholinergika, Betamimetika, Corticosteroiden, SYK-Inhibitoren der Formel **1**, EGFR-Hemmern und PDE4-Antagonisten,
- PDE4-Inhibitoren, Corticosteroiden, EGFR-Hemmern und SYK-Inhibitoren der Formel **1**,
- EGFR-Hemmern, PDE4-inhibitoren und SYK-Inhibitoren der Formel **1**
- EGFR-Hemmern und SYK-Inhibitoren der Formel **1**
- SYK-Inhibitoren der Formel **1**, Betamimetika und Anticholinergika
- Anticholinergika, Betamimetika, Corticosteroiden, PDE4-Inhibitoren und SYK-Inhibitoren der Formel **1**.
Auch die Kombinationen dreier Wirkstoffe je einer der o.g. Verbindungsklassen ist Bestandteil der Erfindung.

Als Betamimetika gelangen hierbei vorzugsweise Verbindungen zur Anwendung, die ausgewählt sind aus der Gruppe bestehend aus Albuterol, Bambuterol, Bitolterol, Broxaterol, Carbuterol, Carmoterol, Indacaterol, Clenbuterol, Fenoterol, Formoterol, Arformoterol, Zinterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmeterol, Salmefamol, Soterenol, Sulphonterol, Tiaramide, Terbutaline, Tolubuterol, CHF-1035, HOKU-81, KUL-1248, 3-(4-{6-[2-Hydroxy-2-(4-hydroxy-3-hydroxymethyl-phenyl)-ethylamino]-hexyloxy}-butyl)-benzyl-sulfonamid, 5-[2-(5,6-Diethyl-indan-2-ylamino)-1-hydroxy-ethyl]-8-hydroxy-1H-quinolin-2-on, 4-Hydroxy-7-[2-{[2-{[3-(2-phenylethoxy)propyl]sulphonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.-butylamino)ethanol, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxy-phenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-(4-Ethoxy-carbonylamino-3-cyano-5-fluorophenyl)-2-(tert.-butylamino)ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Von diesen Betamimetika sind erfindungsgemäß besonders bevorzugt Formoterol, Salmeterol, 3-(4-{6-[2-Hydroxy-2-(4-hydroxy-3-hydroxymethyl-phenyl)-ethylamino]-hexyloxy}-butyl)-benzenesulfoneamide, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxy-phenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 5-[2-(5,6-Diethyl-indan-2-ylamino)-1-hydroxy-ethyl]-8-hydroxy-1H-quinolin-2-on, gegebenenfalls in Form ihrer Racemate, Enantiomere, Diasteromere und gegebenenfalls in Form ihrer pharmakologisch verträglichen Säureadditionssalze, Solvate oder Hydrate.

Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Als Anticholinergika gelangen hierbei vorzugsweise Verbindungen zur Anwendung, die ausgewählt sind aus der Gruppe bestehend aus Tiotropiumsalzen, Oxitropiumsalzen, Flutropiumsalzen, Ipratropiumsalzen, Glycopyrroniumsalzen, Aclidiniumbromid, Trospiumsalzen, 2,2-Diphenylpropionsäuretropenolester-methobromid, 2,2-Diphenylpropionsäurescopinestermethobromid, 2-Fluor-2,2-Diphenylessigsäurescopinester-methobromid, 2-Fluor-2,2-Diphenylessigsäuretropenolester-methobromid, 3,3',4,4'-Tetrafluorbenzilsäuretropenolester-Methobromid, 3,3',4,4'-Tetrafluorbenzilsäurescopinester-Methobromid, 4,4'-Difluorbenzilsäuretropenolester-Methobromid, 4,4'-Difluorbenzilsäurescopinester-Methobromid, 3,3'-Difluorbenzilsäuretropenolester-Methobromid, 3,3'-Difluorbenzilsäurescopinester-Methobromid, 9-Hydroxy-fluoren-9-carbonsäuretropenolester -Methobromid, 9-Fluor-fluoren-9-carbonsäuretropenolester - Methobromid, 9-Hydroxy-fluoren-9-carbonsäurescopinester -Methobromid, 9-Fluor-fluoren-9-carbonsäurescopinester Methobromid, 9-Methyl-fluoren-9-carbonsäuretropenolester Methobromid, 9-Methyl-fluoren-9-carbonsäurescopinester Methobromid, Benzilsäurecyclopropyltropinester-Methobromid, 2,2-Diphenylpropionsäurecyclopropyltropinester -Methobromid, 9-Hydroxy-xanthen-9-carbonsäurecyclopropyltropinesterMethobromid, 9-Methyl-fluoren-9-carbonsäure-cyclopropyltropinester-Methobromid, 9-Methyl-xanthen-9-carbonsäurecyclopropyltropinester - Methobromid, 9-Hydroxy-fluoren-9-carbonsäurecyclopropyltropinester -Methobromid, 4,4'-Difluorbenzilsäuremethylestercyclopropyltropinester -Methobromid, 9-Hydroxy-xanthen-9-carbonsäuretropenolester -Methobromid, 9-Hydroxy-xanthen-9-carbonsäurescopinester Methobromid, 9-Methyl-xanthen-9-carbonsäuretropenolester -Methobromid, 9-Methyl-xanthen-9-carbonsäurescopinester -Methobromid, 9-Ethyl-xanthen-9-carbonsäuretropenolester Methobromid, 9-Difluormethyl-xanthen-9-carbonsäuretropenolester -Methobromid. 9-Hydroxymethyl-xanthen-9-carbonsäurescopinester -Methobromid, gegebenenfalls in Form ihrer Solvate oder Hydrate.

In den vorstehend genannten Salzen stellen die Kationen Tiotropium, Oxitropium, Flutropium, Ipratropium, Glycopyrronium, Aclidinium und Trospium die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodid und Methansulfonat besonders bevorzugt.

Von besonderer Bedeutung ist das Tiotropiumbromid. Im Falle des Tiotropiumbromids enthalten die erfindungsgemäßen Arzneimittelkombinationen dieses bevorzugt in Form des kristallinen Tiotropiumbromid Monohydrats, welches aus der WO 02/30928 bekannt ist. Wird das Tiotropiumbromid in den erfindungsgemäßen Arzneimittelkombinationen in wasserfreier Form eingesetzt, so gelangt bevorzugt das wasserfreie kristalline Tiotropiumbromid zur Anwendung, welches aus der WO 03/000265 bekannt ist.

Als Corticosteroide gelangen hierbei vorzugsweise Verbindungen zur Anwendung, die ausgewählt sind aus der Gruppe bestehend aus Prednisolon, Prednison, Butixocortpropionat, Flunisolid, Beclomethason, Triamcinolon, Budesonid, Fluticason, Mometason, Ciclesonid, Rofleponid, Dexamethason, Betamethason, Deflazacort, RPR-106541, NS-126, 6,9-Difluoro-17-[(2-furanylcarbonyl)oxy]-11-hydroxy-16-methyl-3-oxo-androsta-1,4-dien-17-carbothionsäure (S)-fluoromethylester und 6,9-Difluoro-11-hydroxy-16-methyl-3-oxo-17-propionyloxy-androsta-1,4-dien-17-carbothionsäure (S)-(2-oxo-tetrahydro-furan-3S-yl)ester, gegebenenfalls in Form ihrer Racemate, Enantiomere oder Diastereomere und gegebenenfalls in Form ihrer Salze und Derivate, ihrer Solvate und/oder Hydrate.

Besonders bevorzugt ist das Steroid ausgewählt aus der Gruppe bestehend aus Budesonid, Fluticason, Mometason, Ciclesonid und 6,9-Difluoro-17-[(2-furanylcarbonyl)oxy]-11-hydroxy-16-methyl-3-oxo-androsta-1,4-dien-17-carbothionsäure (S)-fluoromethylester, gegebenenfalls in Form ihrer Racemate, Enantiomere oder Diastereomere und gegebenenfalls in Form ihrer Salze und Derivate, ihrer Solvate und/oder Hydrate.

Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370,N-(3,5-Dichloro-1-oxo-pyridin-4-yl)-4-difluoromethoxy-3-cyclopropylmethoxybenzamid, (-)p-[(4aR*,10bS*)-9-Ethoxy-1,2,3,4,4a,10b-hexahydro-8-methoxy-2-methylbenzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, 9-Cyclopentyl-5,6-dihydro-7-ethyl-3-(2-thienyl)-9H-pyrazolo[3,4-c]-1,2,4-triazolo[4,3-a]pyridin und 9-Cyclopentyl-5,6-dihydro-7-ethyl-3-(tert-butyl)-9H-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugt ist der PDE4-Inhibitor ausgewählt aus der Gruppe bestehend aus Roflumilast, Ariflo (Cilomilast), Arofyllin,AWD-12-281 (GW-842470), 2-Carbomethoxy-4-cyano-4-(3-cyclopropylmethoxy-4-difluoromethoxyphenyl)cyclohexan-1-on, Cis[4-cyano-4-(3-cyclopropylmethoxy-4-difluoromethoxyphenyl)cyclohexan-1-ol], Atizoram, Z-15370, 9-Cyclopentyl-5,6-dihydro-7-ethyl-3-(2-thienyl)-9H-pyrazolo[3,4-c]-1,2,4-triazolo[4,3-a]pyridin und 9-Cyclopentyl-5,6-dihydro-7-ethyl-3-(tert-butyl)-9H-pyrazolo[3,4-c]-1,2,4-triazolo[4,3-a]pyridin gegebenenfalls in Form ihrer Racemate, Enantiomere oder Diastereomere und gegebenenfalls in Form ihrer pharmakologisch verträglichen Säureadditionssalze, Solvate und/oder Hydrate.

Unter Säureadditionssalzen mit pharmakologisch verträglichen Säuren zu deren Bildung die o.g. PDE4-inhibitoren gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321, 1-(((R)-(3-(2-(6,7-Difluoro-2-quinolinyl)ethenyl)phenyl)-3-(2-(2-hydroxy-2-propyl)phenyl)thio)-methylcyclopropan-essigsäure, 1-(((1(R)-3(3-(2-(2,3-Dichlorothieno[3,2-b]pyridin-5-yl)-(E)-ethenyl)phenyl)-3-(2-(1-hydroxy-1-methylethyl)phenyl)propyl)thio)methyl)cyclopropanessigsäure und [2-[[2-(4-tert-Butyl-2-thiazolyl)-5-benzofuranyl]oxymethyl]phenyl]essigsäure gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Besonders bevorzugt ist der LTD4-Antagonist ausgewählt aus der Gruppe bestehend aus Montelukast, Pranlukast, Zafirlukast, MCC-847 (ZD-3523), MN-001 und MEN-91507 (LM-1507) gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Unter Säureadditionssalzen mit pharmakologisch verträglichen Säuren zu deren Bildung die LTD4-Antagonisten gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Unter Salzen oder Derivaten zu deren Bildung die LTD4-antagonisten gegebenenfalls in der Lage sind, werden beispielsweise verstanden : Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin, 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin, 4-[(R)-(1-Phenylethyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}-furan-2-yl)chinazolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-[1-(tert.-butyloxycarbonyl)-piperidin-4-yloxy]-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(methoxymethyl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{trans-4-[(morpholin-4-yl)sulfonylamino]-cyclohexan-1-yloxy}-7-methoxy-chinazolin, 4-[(3-Chlor-4-fluor-phenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(1-aminocarbonylmethyl-piperidin-4-yloxy)-7-methoxy-chinazolin, 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin; 4-{2-[4-(3-Chloro-4-fluoro-phenylamino)-7-methoxy-quinazolin-6-yloxy]-ethyl}-6-methyl-morpholin-2-one; 4-{4-[4-(3-Chloro-2-fluoro-phenylamino)-7-methoxy-quinazolin-6-yloxy]-cyclohexyl}-1-methyl-piperazin-2-one; 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-ethoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Chlor-4-fluor-phenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluor-phenyl)amino]-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxy-acetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxy-acetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluorphenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, [4-[(3-chloro-4-fluoro-phenyl)amino]-6-{[4-(homomorpholin-4-yl)-1-oxo-2-buten-1-yl]amino}-7-[(S)-(tetrahydrofuran-3-yl)oxy]-quinazoline, 4-[(3-Chlor-4-fluor-phenyl)amino]-7-(2-{4-[(S)-(2-oxo-tetrahydrofuran-5-yl)carbonyl]-piperazin-1-yl}-ethoxy)-6-[(vinylcarbonyl)amino]-chinazolin, 4-[(3-Chlor-4-fluor-phenyl)amino]-7-[2-((S)-6-methyl-2-oxo-morpholin-4-yl)-ethoxy]-6-[(vinylcarbonyl)amino]-chinazolin, 4-[(3-Chlor-4-fluor-phenyl)amino]-7-[4-((R)-6-methyl-2-oxo-morpholin-4-yl)-butyloxy]-6-[(vinylcarbonyl)amino]-chinazolin, 4-[(3-Chlor-4-fluor-phenyl)amino]-7-[4-((S)-6-methyl-2-oxo-morpholin-4-yl)-butyloxy]-6-[(vinylcarbonyl)amino]-chinazolin, 4-[(3-Chlor-4-fluor-phenyl)amino]-7-(2-{4-[(S)-(2-oxo-tetrahydrofuran-5-yl)carbonyl]-piperazin-1-yl}-ethoxy)-6-[(vinylcarbonyl)amino]-chinazolin, 4-[(3-Chlor-4-fluor-phenyl)amino]-7-[2-((S)-6-methyl-2-oxo-morpholin-4-yl)-ethoxy]-6-[(vinylcarbonyl)amino]-chinazolin, 4-[(3-Chlor-4-fluor-phenyl)amino]-7-[4-((R)-6-methyl-2-oxo-morpholin-4-yl)-butyloxy]-6-[(vinylcarbonyl)amino]-chinazolin, 4-[(3-Chlor-4-fluor-phenyl)amino]-7-[4-((S)-6-methyl-2-oxo-morpholin-4-yl)-butyloxy]-6-[(vinylcarbonyl)amino]-chinazolin, Cetuximab, Trastuzumab, ABX-EGF, Mab ICR-62, Gefitinib, Canertinib und Erlotinib, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Unter Säureadditionssalzen mit pharmakologisch verträglichen Säuren zu deren Bildung die EGFR-Hemmer gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopamin-Agonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Eine Bezugnahme auf die vorstehend genannten H1-Antihistaminika schließt im Rahmen der vorliegenden Erfindung eine Bezugnahme auf deren gegebenenfalls existierende pharmakologisch verträgliche Säureadditionssalze ein.

Als PAF-Antagonisten gelangen hierbei vorzugsweise Verbindungen zur Anwendung, die ausgewählt sind aus der Gruppe bestehend aus 4-(2-Chlorphenyl)-9-methyl-2-[3(4-morpholinyl)-3-propanon-1-yl]-6H-thieno-[3,2-f]-[1,2,4]triazolo[4,3-a][1,4]diazepin, 6-(2-Chlorphenyl)-8,9-dihydro-1-methyl-8-[(4-morpholinyl)carbonyl]-4H,7H-cyclo-penta-[4,5]thieno-[3,2-f][1,2,4]triazolo[4,3-a][1,4]diazepin. Eine Bezugnahme auf die vorstehend genannten PAF-Antagonisten schließt im Rahmen der vorliegenden Erfindung eine Bezugnahme auf deren gegebenenfalls existierende pharmakologisch verträgliche Säureadditionssalze ein.

Als MRP4-Inhibitoren gelangen hierbei vorzugsweise Verbindungen zur Anwendung, die ausgewählt sind aus der Gruppe bestehend aus *N*-Acetyl-dinitrophenyl-Cysteine, cGMP, Cholate, Diclofenac, Dehydroepiandrosterone 3-glucuronide, Dehydroepiandrosterone 3-sulphate, Dilazep, Dinitrophenyl-S-glutathione, Estradiol 17-beta-glucuronide, Estradiol 3,17-disulphate, Estradiol 3-glucuronide, Estradiol 3-sulphate, Estrone 3-sulphate, Flurbiprofen, Folate, N5-formyl-tetrahydrofolate, Glycocholate, Glycolithocholic acid sulphate, Ibuprofen, Indomethacin, Indoprofen, Ketoprofen, Lithocholic acid sulphate, Methotrexate, MK571 ((*E*)-3-[[[3-[2-(7-Chloro-2-quinolinyl)ethenyl]phenyl]-[[3-dimethylamino)-3-oxopropyl]thio]methyl]thio]-propanoic acid), alpha-Naphthyl-beta-D-glucuronide, Nitrobenzyl mercaptopurine riboside, Probenecid, PSC833, Sildenafil, Sulfinpyrazone, Taurochenodeoxycholate, Taurocholate, Taurodeoxycholate, Taurolithocholate, Taurolithocholic acid sulphate, Topotecan, Trequinsin und Zaprinast, Dipyridamol, gegebenenfalls in Form ihrer Racemate, Enantiomere, Diastereomere und ihrer pharmakologisch verträglichen Säure-Additionssalze und Hydrate.

Stärker bevorzugt bezieht sich die Erfindung auf die Verwendung von MRP4-Inhibitoren zur Herstellung einer pharmazeutischen Zusammensetzung zur Behandlung von Atemwegserkrankungen enthaltend die erfindungsgemäßen SYK-Inhibitoren und MRP4-Inhibitoren, wobei die MRP4-Inhibitoren vorzugsweise ausgewählt sind aus der Gruppe bestehend aus
Dehydroepiandrosterone 3-sulphate, Estradiol 3,17-disulphate, Flurbiprofen, Indomethacin, Indoprofen, MK571, Taurocholate, gegebenenfalls in Form ihrer Racemate, Enantiomere, Diastereomere und ihrer pharmakologisch verträglichen Säure-Additionssalze und Hydrate. Die Trennung von Enantiomeren aus den Racematen kann durch bekannte Verfahren nach dem Stand der Technik durchgeführt werden (z.B. durch Chromatographie an chiralen Phasen etc.).

Mit Säure-Additionssalzen mit pharmakologisch verträglichen Säuren sind z.B. Salze ausgewählt aus der Gruppe bestehend aus Hydrochloriden, Hydrobromiden, Hydroiodiden, Hydrosulphaten, Hydrophosphaten, Hydromethanesulphonaten, Hydronitraten, Hydromaleaten, Hydroacetaten, Hydrobenzoaten, Hydrocitraten, Hydrofumaraten, Hydrotartraten, Hydrooxalaten, Hydrosuccinaten, Hydrobenzoaten and Hydro-*p*-toluenesulphonaten, vorzugsweise Hydrochloride, Hydrobromide, Hydrosulphate, Hydrophosphate, Hydrofumarate and Hydromethanesulphonate gemeint.

Ein weiterer Gegenstand der Erfindung sind pharmazeutische Zubereitungen, die Dreifachkombinationen von den erfindungsgemäßen SYK-Inhibitoren, von MRP4-Inhibitoren und einer weiteren aktiven Substanz wie z.B. einem Anticholinergikum, einem PDE4-Inhibitor, einem Steroid, einem LTD4-Antagonist oder einem Betamimetikum enthalten, sowie deren Herstellung und deren Verwendung zur Behandlung von Atemwegserkrankungen.

Als iNOS-Inhibitoren gelangen hierbei vorzugsweise Verbindungen zur Anwendung, die ausgewählt sind aus der Gruppe bestehend aus: S-(2-Aminoethyl)isothioharnstoff, Aminoguanidin, 2-Aminomethylpyridin, AMT, L-Canavanin, 2-Iminopiperidin, S-Isopropylisothioharnstoff, S-Methylisothioharnstoff, S-Ethylisothioharnstoff, S-Methyltiocitrullin, S-Ethylthiocitrullin, L-NA (N^{ω}-Nitro-L-arginin), L-NAME (N^{ω}-Nitro-L-argininmethylester), L-NMMA (N^{G}-Monomethyl-L-arginin), L-NIO (N^{ω}-Iminoethyl-L-ornithin), L-NIL (N^{ω}-Iminoethyl-lysin), (S)-6-Acetimidoylamino-2-amino-hexanoic acid (1*H*-tetrazol-5-yl)-amid (SC-51) (J. Med. Chem. 2002, 45, 1686-1689), 1400W, (S)-4-(2-Acetimidoylamino-ethylsulfanyl)-2-amino-buttersäure (GW274150) (Bioorg. Med. Chem. Lett. 2000, 10, 597-600), 2-[2-(4-Methoxy-pyridin-2-yl)-ethyl]-3*H*-imidazo[4,5-b]pyridin (BYK191023) (Mol. Pharmacol. 2006, 69, 328-337), 2-((R)-3-Amino-1-phenyl-propoxy)-4-chlor-5-fluorbenzonitril (WO 01/62704), 2-((1 R,3S)-3-Amino-4-hydroxy-1-thiazol-5-yl-butylsulfanyl)-6-trifluoromethyl-nicotinonitril (WO 2004/041794), 2-((1R,3S)-3-Amino-4-hydroxy-1-thiazol-5-yl-butylsulfanyl)-4-chlor-benzonitril (WO 2004/041794), 2-((1R,3S)-3-Amino-4-hydroxy-1-thiazol-5-yl-butylsulfanyl)-5-chlor-benzonitril (WO 2004/041794), (2S,4R)-2-Amino-4-(2-chlor-5-trifluoromethyl-phenylsulfanyl)-4-thiazol-5-yl-butan-1-ol (WO 2004/041794), 2-((1R,3S)-3-Amino-4-hydroxy-1-thiazol-5-yl-butylsulfanyl)-5-chlor-nicotinonitril (WO 2004/041794), 4-((S)-3-Amino-4-hydroxy-1-phenyl-butylsulfanyl)-6-methoxy-nicotinonitril (WO 02/090332), substituierte 3-Phenyl-3,4-dihydro-1-isoquinolinamin wie z.B. AR-C102222 (J. Med. Chem. 2003, 46, 913-916), (1S,5S,6R)-7-Chlor-5-methyl-2-aza-bicyclo[4.1.0]hept-2-en-3-ylamin (ONO-1714) (Biochem. Biophys. Res. Commun. 2000, 270, 663-667), (4R,5R)-5-Ethyl-4-methyl-thiazolidin-2-ylideneamin (Bioorg. Med. Chem. 2004, 12, 4101), (4R,5R)-5-Ethyl-4-methyl-selenazolidin-2-ylideneamin (Bioorg. Med. Chem. Lett. 2005, 15, 1361), 4-Aminotetrahydrobiopterin (Curr. Drug Metabol. 2002, 3, 119-121), (E)-3-(4-Chlor-phenyl)-*N*-(1-{2-oxo-2-[4-(6-trifluormethyl-pyrimidin-4-yloxy)-piperidin-1-yl]-ethylcarbamoyl}-2-pyridin-2-yl-ethyl)-acrylamid (FR260330) (Eur. J. Pharmacol. 2005, 509, 71-76), 3-(2,4-Difluorphenyl)-6-[2-(4-imidazol-1-ylmethyl-phenoxy)-ethoxy]-2-phenyl-pyridin (PPA250) (J. Pharmacol. Exp. Ther. 2002, 303, 52-57), 3-{[(Benzo[1,3]dioxol-5-ylmethyl)-carbamoyl]-methyl}-4-(2-imidazol-1-yl-pyrimidin-4-yl)-piperazin-1-carbonsäuremethylester (BBS-1) (Drugs Future 2004, 29, 45-52), (R)-1-(2-Imidazol-1-yl-6-methyl-pyrimidin-4-yl)-pyrrolidin-2-carbonsäure (2-benzo[1,3]dioxol-5-yl-ethyl)-amid (BBS-2) (Drugs Future 2004, 29, 45-52) und deren pharmazeutischen Salze, Prodrugs oder Solvate.

Als iNOS-Inhibitoren im Rahmen der vorliegenden Erfindung können weiterhin antisense-Oligonucleotide, insbesondere solche antisense-Oligonucleotide, die iNOS-kodierende Nukleinsäuren binden, eingesetzt werden. Z.B. werden in WO 01/52902 antisense-Oligonucleotide, insbesondere antisense-Oligonucleotide, die iNOS kodierende Nukleinsäuren binden, zur Modulierung der Expression von iNOS beschrieben. Solche iNOS-antisense-Oligonucleotide wie insbesondere in WO 01/52902 beschrieben können daher auch aufgrund ihrer ähnlichen Wirkung wie die iNOS-Inhibitoren mit den PDE4-Inhibitoren der vorliegenden Erfindung kombiniert werden.

### 8. DARREICHUNGSFORMEN

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0, 1 bis 90 Gew.-%, bevorzugt 0, 5 bis 50 Gew.-% der Gesamtzusammensetzung liegen, d.h. in Mengen die ausreichend sind, um den unten angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel **1** gemäß der obigen bevorzugten Ausführungsformen.

Besonders bevorzugt ist es, wenn die Verbindungen der Formel **1** oral verabreicht werden, besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie
Natriumcarboxymethylcellulose, Netzmittel, beispielsweise Kondensationsprodukte von Fettalkoholen mit Ethylenoxid, oder Schutzstoffe, wie *p*-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.
Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Ebenfalls bevorzugt ist es, wenn die Verbindungen der Formel **1** inhalativ verabreicht werden, besonders bevorzugt ist es, wenn die Verabreichung ein oder zweimal täglich erfolgt. Hierzu müssen die Verbindungen der Formel **1** in inhalierbaren Darreichungsformen bereitgestellt werden. Als inhalierbare Darreichungsformen kommen Inhalationspulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### Inhalationspulver

Sind die Verbindungen der Formel **1** im Gemisch mit physiologisch unbedenklichen Hilfsstoffen enthalten, können zur Darstellung der erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung. Verfahren zur Herstellung der erfindungsgemäßen Inhalationspulver durch Mahlen und Mikronisieren sowie durch abschließendes Mischen der Bestandteile sind aus dem Stand der Technik bekannt.

### Treibgashaltige Inhalationsaerosole

Die im Rahmen der erfindungsgemäßen Verwendung einsetzbaren treibgashaltigen Inhalationsaerosole können die Verbindungen der Formel **1** im Treibgas gelöst oder in dispergierter Form enthalten. Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind fluorierte Alkanderivate ausgewählt aus TG134a (1, 1, 1, 2-Tetrafluorethan), TG227 (1, 1, 1, 2, 3, 3, 3-Heptafluorpropan) und Mischungen derselben. Die im Rahmen der erfindungsgemäßen Verwendung einsetzbaren treibgashaltigen Inhalationsaerosole können ferner weitere Bestandteile wie Co-Solventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

### Treibgasfreie Inhalationslösungen

Die erfindungsgemäße Verwendung von Verbindungen der Formel **1** erfolgt bevorzugt zur Herstellung von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Die Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Den im Rahmen der erfindungsgemäßen Verwendung einsetzbaren treibgasfreien Inhalationslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.
Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien. Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine. Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration.

Für die oben beschriebenen Behandlungsformen werden gebrauchsfertige Packungen eines Medikaments zur Behandlung von Atemwegserkrankungen, beinhaltend eine beigelegte Beschreibung, welche beispielsweise die Worte Atemwegserkrankung, COPD oder Asthma enthalten, ein Naphthyridin nach Formel **1** und ein oder mehrere Kombinationspartner ausgewählt aus der oben beschriebenen Gruppe, bereit gestellt.

## Patentansprüche

1. Verbindungen der Formel **1** 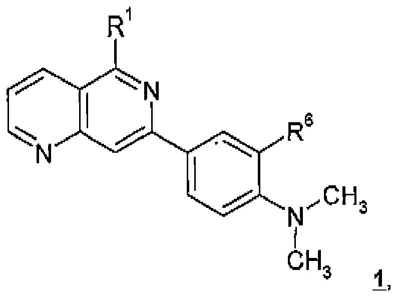 wobei
**R¹** ein Rest **A** ausgewählt aus der Gruppe bestehend aus -O-R³, -NR³R⁴,
**R³** ein Rest ist ausgewählt aus der Gruppe bestehend aus -C₁₋₆-Alkyl, -C₁₋₆-Fluoroalkyl, -(C₁₋₅-Alkyl)-(OH, -C₆₋₁₀-Aryl, -(C₁₋₄-Alkylen)-(C₆₋₁₀-Aryl), -Ethenyl, -(C₁₋₄-Alkylen)-(Ethen), - Ethinyl, -(C₁₋₄-Alkylen)-(Ethin), -(C₁₋₄-Alkylen)-(Ethin)-NH₂, -(C₁₋₄-Alkylen)-(Ethin)-(C₁₋₄-Alkylen)-NH₂, -NH(C₁₋₃-Alkyl), -(C₁₋₄-Alkylen)-NH(C₁₋₃-Alkyl), -CHOH-(C₁₋₄-Alkylen)-NH₂, -(C₁₋₄-Alkylen)-CHOH-(C₁₋₄-Alkylen)-NH₂, -(C₁₋₄-Alkylen)-CHOH-NH₂, -CHOH-NH₂, mono- oder bicyclisches, gesättigtes oder teilweise gesättigtes -C₃₋₁₀-Cycloalkyl, mono- oder bicyclisches, gesättigtes oder teilweise gesättigtes -(C₁₋₄-Alkylen)-C₃₋₁₀Cycloalkyl, -(Het), -(C₁₋₄-Alkylen)-(Het), -(Hetaryl), und -(C₁₋₄-Alkylen)-(Hetaryl),
wobei dieser Rest
gegebenenfalls mit einem oder mehreren Resten unabhängig voneinander ausgewählt aus der Gruppe bestehend aus H, -OH, -Oxo, -COOH, -Halogen, -C₁₋₃-Alkyl, -C₁₋₃-Haloalkyl, -C₁₋₃-Alkyl-OH, -C₃₋₇-Cycloalkyl, -O-(C₁₋₄-Alkyl), -NH(C₁₋₄-Alkyl), -(C₁₋₄-Alkylen)-NH(C₁₋₄-Alkyl), - N(C₁₋₄-Alkyl)₂, -(C₁₋₄-Alkylen)-N(C₁₋₄-Alkyl)₂, -NH-CONH₂, -(C₁₋₄-Alkylen)-NH-CO-NH₂, -CO-NH₂, -(C₁₋₄-Alkylen)-CO-NH₂, -CO-NH(C₁₋₃-Alkyl), -(C₁₋₄-Alkylen)-CO-NH(C₁₋₃-Alkyl), -CO-N(C₁₋₃-Alkyl)₂, -(C₁₋₄-Alkylen)-CO-N(C₁₋₃-Alkyl)₂, -NH-(CO)ₘ-NH₂, -NH-(C₁₋₄-Alkylen)-(CO)ₘ-NH₂, -NH-(CO)ₘ-NH(C₁₋₃-Alkyl), -NH-(C₁₋₄-Alkylen)-(CO)ₘ-NH(C₁₋₃-Alkyl), -NH-(CO)ₘ-N(C₁₋₃-Alkyl)₂, -NH-(C₁₋₄-Alkylen)-(CO)ₘ-N(C₁₋₃-Alkyl)₂, -O-(C₂₋₄-Alkylen)-NH₂, -O-(C₂₋₄-Alkylen)-NH(C₁₋₃-Alkyl), -O-(C₂₋₄-Alkylen)-N(C₁₋₃-Alkyl)₂, -NH-CO-(C₁₋₃-Alkyl), -(C₁₋₄-Alkylen)-NH-CO-(C₁₋₃-Alkyl), C₃₋₅-Cycloalkyl, -SO₂-(C₁₋₄-Alkyl), - NH-(Hetaryl), -NH-(C₁₋₄-Alkylen)-(Hetaryl), -(Het) und -(C₁₋₄-Alkylen)-(Het) substituiert sein kann,
wobei (Het) für einen drei- bis zehngliedrigen, gesättigten oder teilweise gesättigten, mono- oder bicyclischen, gegebenenfalls mit 1-3 Resten ausgewählt aus C₁₋₃-Alkyl, Halogen, CH₂-NH₂, NH₂, OH, CO-NH₂ und Oxo substituierten Heterocyclus steht, der 1, 2 oder 3 Heteroatome unabhängig voneinander ausgewählt aus der Gruppe bestehend aus N, O und S enthält,
und
wobei **(Hetaryl)** für ein fünf- bis zehngliedriges, mono- oder bicyclisches, gegebenenfalls mit 1, 2 oder 3 Resten ausgewählt aus C₁₋₃-Alkyl, Halogen, CH₂-NH₂, NH₂, OH, CO-NH₂ und Oxo substituiertes Heteroaryl steht, das 1-3 Heteroatome unabhängig voneinander ausgewählt aus der Gruppe bestehend aus N, O und S enthält,
m = 0 oder 1
R⁴ H, Methyl oder Ethyl
R⁶ Methyl,
sowie pharmazeutisch verträgliche Salze davon.

2. Verbindungen der Formel **1** nach Anspruch 1,
wobei R¹ -O-R³ oder -NR³R⁴ ist
und wobei
**(Het)** für einen drei- bis siebengliedrigen, gesättigten oder teilweise gesättigten, monocyclischen, gegebenenfalls mit 1-3 Resten ausgewählt aus Methyl, Ethyl Propyl, Isopropyl, F, Cl, Br, CH₂-NH₂, NH₂, OH, CO-NH₂ und Oxo substituierten Heterocyclus steht, der 1, 2 oder 3 Heteroatome unabhängig voneinander ausgewählt aus der Gruppe bestehend aus N, O und S enthält,
und wobei
**(Hetaryl)** für ein fünf- bis sechsgliedriges, monocyclisches, gegebenenfalls mit 1, 2 oder 3 Resten ausgewählt aus Methyl, Ethyl Propyl, Isopropyl, F, Cl. Br, CH₂-NH₂, NH₂, OH, CO-NH₂ und Oxo substituiertes Heteroaryl steht, das 1-3 Heteroatome unabhängig voneinander ausgewählt aus der Gruppe bestehend aus N, O und S enthält,
sowie pharmazeutisch verträgliche Salze davon.

3. Verbindungen der Formel **1** nach einem der Ansprüche 1 bis 2,
wobei **R¹** ausgewählt ist aus der Gruppe bestehend aus 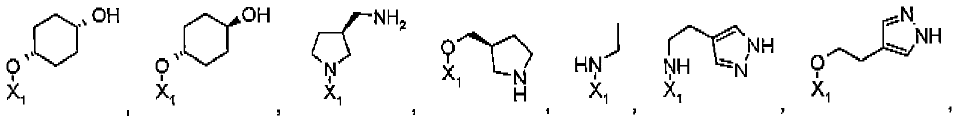 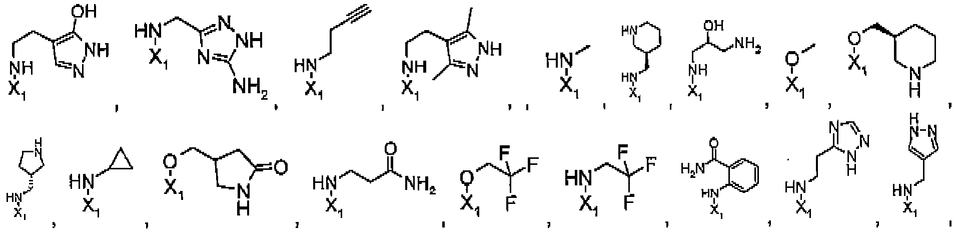 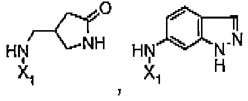 und,
wobei X₁ den Anknüpfungspunkt von **R¹** an die Struktur der Formel **1** bezeichnet und
und wobei **R⁶** Methyl ist.
sowie pharmazeutisch verträgliche Salze davon.

4. Verbindungen nach einem der Ansprüche 1 bis 3 als Arzneimittel.

5. Verwendung von Verbindungen nach einem der Ansprüche 1 bis 3, zur Herstellung eines Medikaments zur Behandlung von Erkrankungen ausgewählt aus der Gruppe bestehend aus Allergische Rhinitis, Asthma, COPD, Adultes respiratorisches Distress Syndrom, Bronchitis, B-Zell-Lymphome, Dermatitis und Kontakt Dermatitis, allergische Dermatitis, allergische Rhinokonjunktivitis, rheumatoide Arthritis, Anti-Phospholipid Syndrom, Berger-Krankheit, Evans Syndrom, Kolitis ulcerosa, allergische Antikörper-basierende Glomerulonephritis, Granulozytopenie, Goodpasture Syndrom, Hepatitis, Henoch-Schönlein Purpura, Hypersensitivitätsvaskulitis, Immunhämolytische Anämie, idiopathische thrombozytopenische Purpura, Kawasaki Syndrom, Allergische Konjunktivitis, Lupus erythematodes, Mantelzell-Lymphom, Neutropenie, Nicht familiäre lateral Sklerose, Morbus Crohn, multiple Sklerose, Myastenia Gravis, Myelodystplastisches Syndrom, Osteoporose, Osteolytische Erkrankungen,Osteopenie, Psiorasis, Sjögren Syndrome, Sklerodermie, T-Zeil-Lymphom, Urtikaria / Angioödema, Wegener-Granulomatose und Zöliakie.

6. Verwendung nach Anspruch 5, wobei die Erkrankung ausgewählt ist aus der Gruppe bestehend aus Asthma, COPD, allergische Rhinitis, Adultes respiratorisches Distress Syndrom, Bronchitis, allergische Dermatitis, Kontakt Dermatitis, idiopathische thrombozytopenische Purpura, rheumatoide Arthritis und allergische Rhinokonjunktivitis.

7. Verwendung von Verbindungen nach einem der Ansprüche 5 oder 6, wobei die Erkrankung ausgewählt ist aus der Gruppe bestehend aus Asthma, COPD, allergische Rhinitis, allergische Dermatitis und rheumatoide Arthritis.

8. Pharmazeutische Formulierungen, **gekennzeichnet durch** den Gehalt an einer oder mehreren Verbindungen der Formel **1** nach einem der Ansprüche 1 bis 3.

9. Pharmazeutische Formulierungen **gekennzeichnet durch** den Gehalt an einer oder mehreren Verbindungen der Formel **1,** nach einem der Ansprüche 1 bis 3 in Kombination mit einem Wirkstoff ausgewählt aus der Gruppe bestehend aus Betamimetika, Corticosteroiden, PDE4-Inhibitoren, EGFR-Hemmern und LTD4-Antagonisten, CCR3-Inhibitoren, iNOS-Inhibitoren und SYK-Inhibitoren.

10. Verbindungen ausgewählt aus der Gruppe bestehend aus 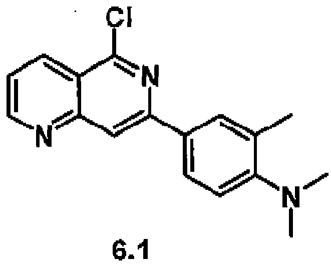 und

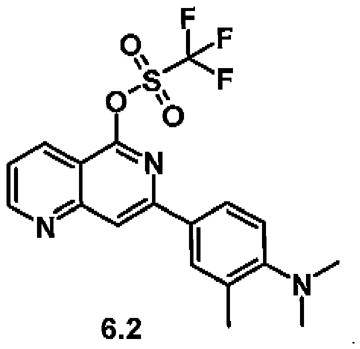

## Claims

1. Compounds of formula **1** 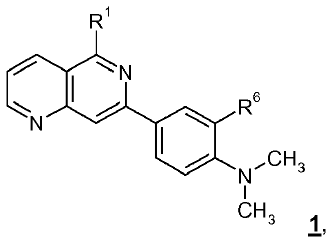 wherein
**R¹** denotes a group **A** selected from among -O-R³, -NR³R⁴,
**R³** is a group selected from among -C₁₋₆-alkyl, -C₁₋₆-fluoroalkyl, -(C₁₋₅-alkyl)-OH, -C₆₋₁₀-aryl, -(C₁₋₄-alkylene)-(C₆₋₁₀₋aryl), -ethenyl, -(C₁₋₄-alkylene)-(ethene), -ethynyl, -(C₁₋₄-alkylene)-(ethyne), -(C₁₋₄-alkylene)-(ethyne)-NH₂, -(C₁₋₄-alkylene)-(ethyne)-(C₁₋₄-alkylene)-NH₂, -NH(C₁₋₃-alkyl), -(C₁₋₄-alkylene)-NH(C₁₋₃-alkyl), -CHOH-(C₁₋₄-alkylene)-NH₂, -(C₁₋₄-alkylene)-CHOH-(C₁₋₄-alkylene)-NH₂, -(C₁₋₄-alkylene)-CHOH-NH₂, -CHOH-NH₂, mono- or bicyclic, saturated or partly saturated -C₃₋₁₀-cycloalkyl, mono- or bicyclic, saturated or partly saturated -(C₁₋₄-alkylene)-C₃₋₁₀-cycloalkyl, -(het), -(C₁₋₄-alkylene)-(het), -(hetaryl), and -(C₁₋₄-alkylene)-(hetaryl),
wherein this group may optionally be substituted by one or more groups selected independently of one another from among H, -OH, -oxo, -COOH, -halogen, -C₁₋₃-alkyl, -C₁₋₃-haloalkyl, -C₁₋₃-alkyl-OH, -C₃₋₇-cycloalkyl, -O-(C₁₋₄-alkyl), -NH(C₁₋₄-alkyl), -(C₁₋₄-alkylene)-NH(C₁₋₄-alkyl), -N(C₁₋₄-alkyl)₂, -(C₁₋₄-alkylene)-N(C₁₋₄-alkyl)₂, -NH-CO-NH₂, -(C₁₋₄-alkylene)-NH-CO-NH₂, -CO-NH₂, -(C₁₋₄-alkylene)-CO-NH₂, -CO-NH(C₁₋₃-alkyl), -(C₁₋₄-alkylene)-CO-NH(C₁₋₃-alkyl), -CO-N(C₁₋₃-alkyl)₂, -(C₁₋₄-alkylene)-CO-N(C₁₋₃-alkyl)₂, -NH-(CO)ₘ-NH₂, -NH-(C₁₋₄-alkylene)-(CO)ₘ-NH₂, -NH-(CO)ₘ-NH(C₁₋₃-alkyl), -NH-(C₁₋₄-alkylene)-(CO)ₘ-NH(C₁₋₃-alkyl),-NH-(CO)ₘ-N(C₁₋₃-alkyl)₂, -NH-(C₁₋₄-alkylene)-(CO)ₘ-N(C₁₋₃-alkyl)₂, -O-(C₂₋₄-alkylene)-NH₂, -O-(C₂₋₄-alkylene)-NH(C₁₋₃-alkyl), -O-(C₂₋₄-alkylene)-N(C₁₋₃-alkyl)₂, -NH-CO-(C₁₋₃-alkyl), -(C₁₋₄-alkylene)-NH-CO-(C₁₋₃-alkyl), C₃₋₅-cycloalkyl, -SO₂-(C₁₋₄-alkyl), -NH-(hetaryl), -NH-(C₁₋₄-alkylene)-(hetaryl), -(het) and -(C₁₋₄-alkylene)-(het),
wherein **(het)** denotes a three- to ten-membered, saturated or partly saturated, mono- or bicyclic heterocyclic group, optionally substituted by 1-3 groups selected from C₁₋₃-alkyl, halogen, CH₂-NH₂, NH₂, OH, CO-NH₂ and oxo, which contains 1, 2 or 3 heteroatoms selected independently of one another from among N, O and S,
and
wherein **(hetaryl)** denotes a five- to ten-membered, mono- or bicyclic heteroaryl, optionally substituted by 1, 2 or 3 groups selected from C₁₋₃-alkyl, halogen, CH₂-NH₂, NH₂, OH, CO-NH₂ and oxo, which contains 1-3 heteroatoms selected independently of one another from among N, O and S,
**m** = 0 or 1
**R⁴** denotes H, methyl or ethyl
**R⁶** denotes methyl,
as well as pharmaceutically acceptable salts thereof.

2. Compounds of formula **1** according to claim 1, wherein
wherein **R¹** is -O-R³ or -NR³R⁴
and wherein
**(het)** denotes a three- to seven-membered, saturated or partly saturated, monocyclic heterocyclic group, optionally substituted by 1-3 groups selected from methyl, ethyl propyl, isopropyl, F, Cl, Br, CH₂-NH₂, NH₂, OH, CO-NH₂ and oxo, which contains 1, 2 or 3 heteroatoms selected independently of one another from among N, O and S,
and wherein
**(hetaryl)** denotes a five- to six-membered, monocyclic heteroaryl, optionally substituted by 1, 2 or 3 groups selected from methyl, ethyl propyl, isopropyl, F, Cl, Br, CH₂-NH₂, NH₂, OH, CO-NH₂ and oxo, which contains 1-3 heteroatoms selected independently of one another from among N, O and S,
as well as pharmaceutically acceptable salts thereof.

3. Compounds of formula **1** according to one of claims 1 to 2,
wherein **R¹** is selected from among 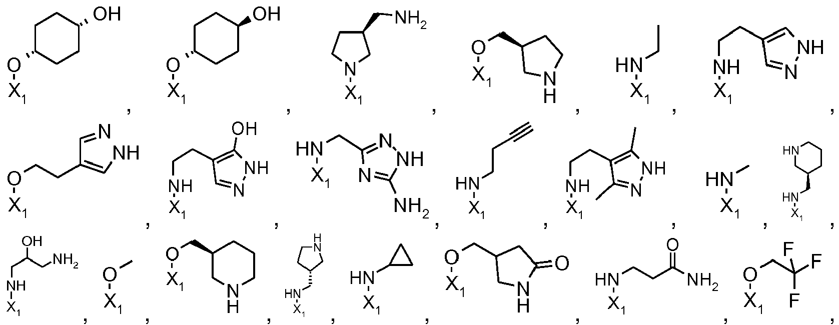 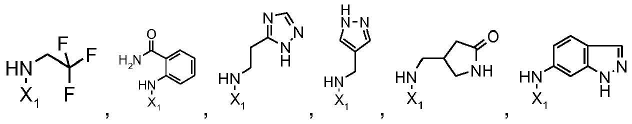 and
wherein **X₁** denotes the point of attachment of **R¹** to the structure of formula **1** and
wherein **R⁶** is methyl,
as well as pharmaceutically acceptable salts thereof.

4. Compounds according to one of claims 1 to 3 for use as a medicament.

5. Use of compounds according to one of claims 1 to 3, for preparing a medicament for the treatment of diseases selected from the group consisting of allergic rhinitis, asthma, COPD, adult respiratory distress syndrome, bronchitis, B-cell lymphoma, dermatitis and contact dermatitis, allergic dermatitis, allergic rhinoconjunctivitis, rheumatoid arthritis, anti-phospholipid syndrome, Berger's disease, Evans syndrome, ulcerative colitis, allergic antibody-based glomerulonephritis, granulocytopenia, Goodpasture syndrome, hepatitis, Henoch-Schönlein purpura, hypersensitivity vasculitis, immunohaemolytic anaemia, idiopathic thrombocytopenic purpura, Kawasaki syndrome, allergic conjunctivitis, lupus erythematodes, capsule cell lymphoma, neutropenia, non-familial lateral sclerosis, Crohn's disease, multiple sclerosis, myasthenia gravis, myelodysplastic syndrome, osteoporosis, osteolytic diseases, osteopenia, psoriasis, Sjögren's syndrome, scleroderma, T-cell lymphoma, urticaria / angiooedema, Wegener's granulomatosis and coeliac disease.

6. Use according to claim 5, wherein the disease is selected from the group consisting of asthma, COPD, allergic rhinitis, adult respiratory distress syndrome, bronchitis, allergic dermatitis, contact dermatitis, idiopathic thrombocytopenic purpura, rheumatoid arthritis and allergic rhinoconjunctivitis.

7. Use of compounds according to one of Claims 5 or 6, wherein the disease is selected from among asthma, COPD, allergic rhinitis, allergic dermatitis and rheumatoid arthritis.

8. Pharmaceutical formulations, **characterised in that** they contain one or more compounds of formula **1** according to one of Claims 1 to 3.

9. Pharmaceutical formulations, **characterised in that** they contain one or more compounds of formula **1** according to one of Claims 1 to 3 in combination with an active substance selected from among betamimetics, corticosteroids, PDE4-inhibitors, EGFR-inhibitors and LTD4-antagonists, CCR3-inhibitors, iNOS-inhibitors and SYK-inhibitors.

10. Compounds selected from among 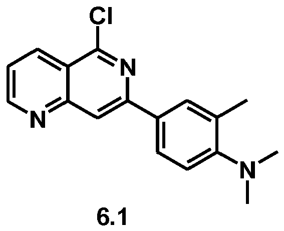 and

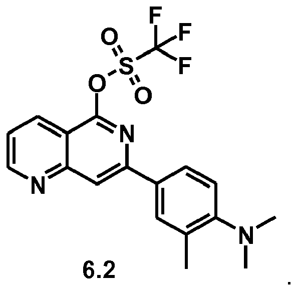

## Revendications

1. Composés de formule 1 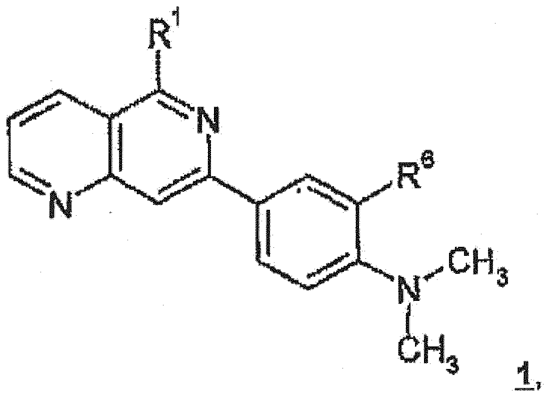 dans laquelle
R¹ représente un radical A choisi dans le groupe comprenant -O-R³, -NR³R⁴,
R³ représente un radical choisi dans le groupe comprenant un groupe -C₁-₆-alkyle, -C₁-₆-fluoroalkyle, - (C₁₋₅-alkyle) -OH, -C₆₋₁₀-aryle, - (C₁₋₄-alkylène) - (C₆₋₁₀-aryle), -éthényle, -(C₁-₄-alkylène)-(éthène), éthinyle, -(C₁₋₄-alkylène)-(éthine), -(C₁-₄-alkylène)-(éthine)-NH₂, - (C₁-₄-alkylène) - (éthine) - (C₁-₄-alkylène) -NH₂, -NR(C₁-₃-alkyle), -(C₁-₄-alkylène)-NH(C₁-₃-alkyle), -CHOH-(C₁-₄-alkylène) -NH₂, -(C₁-₄-alkylène) -CHOH-(C₁-₄-alkylène)-NH₂, -(C₁-₄-alkylène)-CHOH-NH₂, -CHOH-NH₂, -C₃-₁₀-cycloalkyle monocyclique ou bicyclique saturé ou partiellement saturé, -(C₁-₄-alkylène)-C₃-₁₀-cycloalkyle monocyclique ou bicyclique, saturé ou partiellement saturé, -(het), -(C₁-₄-alkylène)-(het), -(hétaryle) et -(C₁-₄-alkylène)-(hétaryle),
dans laquelle ce radical
peut être substitué éventuellement par un ou plusieurs radicaux choisis indépendamment les uns des autres dans le groupe comprenant H, -OH, -oxo, -COOH, -halogéno, - C₁-₃-alkyle, C₁-₃-halogénoalkyle, C₁-₃-alkyle-OH, -C₃-₇-cycloalkyle, -O- (C₁-₄-alkyle), -NH (C₁-₄-alkyle), - (C₁-₄-alkylène)-NH (C₁-₄-alkyle) , N(C₁-₄-alkyle)₂, -(C₁-₄-alkylène)-N(C₁-₄-alkyle)₂, -NH-CO-NH₂, -(C₁-₄-alkylène)-NH-CO-NH₂, -CO-NH₂, -(C₁-₄-alkyléne)-CO-NH₂, -CO-NH(C₁-₃-alkyle), -(C₁-₄-alkylène)-CO-NH(C₁-₃-alkyle), -CO-N(C₁-₃-allyle)₂, -(C₁-₄-alkylène)-CO-N(C₁-₃-alkyle)₂, -NH(CO)ₘ-NH₂, -NH-(C₁-₄-alkylène)-(CO)ₘ-NH₂, -NH-(CO)ₘ-NH(C₁-₃-alkyle), -NH-(C₁-₄-alkylène)-(CO)ₘ-NH(C₁-₃-alkyle), -NH-(CO)ₘ-N(C₁-₃-alkyle)₂, -NH-(C₁-₄-alkylène)-(CO)ₘ-N(C₁-₃-allyle)₂, -O-(C₂-₄-alkylène)-NH₂, -O-(C₂-₄-alylène)-NH(C₁-₃-alkyle), -O- (C₂-₄-alkylène) -N(C₁-₃-alkyle)₂, -NH-CO- (C₁-₃-alkyle), - (C₁-₄-alkylène) -NH-CO- (C₁-₃-alkyle), C₃-₅-cycloalkyle, -SO₂-(C₁-₄-alkyle), -NH-(hétaryle), -NH-(C₁-₄-alkylène)-(hétaryle), -(het) et - (C₁-₄-alkylène)-(het),
dans laquelle (het) représente un hétérocycle de trois à dix chaînons, saturé ou partiellement saturé, monocyclique ou bicyclique, substitué éventuellement par 1 à 3 radicaux choisis parmi les groupes C₁-₃-alkyle, halogéno, CH₂-NH₂, NH₂, OH, CO-NH₂ et oxo, qui contient 1, 2 ou 3 hétéroatomes choisis indépendamment les uns des autres dans le groupe comprenant N, O et S, et
dans laquelle le groupe (hétaryle) représente un groupe hétéroaryle de cinq à dix chaînons, monocyclique ou bicyclique, substitué éventuellement par 1, 2 ou 3 radicaux choisis parmi les groupes C₁-₃-alkyle, halogéno, CH₂-NH₂, NH₂, OH, CO-NH₂ et oxo, qui contient 1 à 3 hétéroatomes choisis indépendamment les uns des autres dans le groupe comprenant N, O et S,
m = 0 ou 1,
R⁴ représente H, un groupe méthyle ou éthyle,
R⁶ représente un groupe méthyle,
et leurs sels pharmaceutiquement acceptables.

2. Composés de formule 1 selon la revendication 1, dans laquelle R¹ est -O-R³ ou -NR³R⁴
et dans laquelle
(het) représente un hétérocycle de trois à sept chaînons, saturé ou partiellement saturé, monocyclique, substitué éventuellement par 1 à 3 radicaux choisis parmi les groupes méthyle, éthyle, propyle, isopropyle, F, Cl, Br, CH₂-NH₂, NH₂, OH, CO-NH₂ et oxo, qui contient 1, 2 ou 3 hétéroatomes choisis indépendamment les uns des autres dans le groupe comprenant N, O et S,
et dans laquelle
(hétaryle) représente un groupe hétéroaryle de cinq à six chaînons, monocyclique, éventuellement substitué par 1, 2 ou 3 radicaux choisis parmi les groupes méthyle, éthyle, propyle, isopropyle, F, Cl, Br, CH₂-NH₂, NH₂, OH, CO-NH₂ et oxo, qui contient 1 à 3 hétéroatomes choisis indépendamment les uns des autres dans le groupe comprenant N, O et S,
et leurs sels pharmaceutiquement acceptables.

3. Composés de formule 1 selon l'une des revendications 1 à 2,
dans laquelle R¹ est choisi dans le groupe comprenant 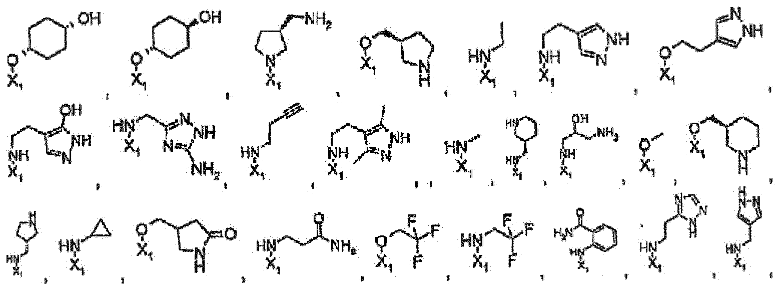 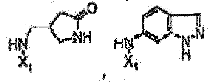 et, dans laquelle X₁ désigne le point de liaison de R¹ à la structure de formule 1 et
dans laquelle M⁶ est un groupe méthyle,
et leurs sels pharmaceutiquement acceptables.

4. Composés selon l'une des revendications 1 à 3 comme médicaments.

5. Utilisation de composés selon l'une des revendications 1 à 3, pour la production d'un médicament pour le traitement de maladies choisies dans le groupe comprenant la rhinite allergique, l'asthme, la BPCO, le syndrome de détresse respiratoire de l'adulte, la bronchite, le lymphome à cellules B, la dermatite et la dermatite de contact, la dermatite allergique, la rhinoconjonctivite allergique, l'arthrite rhumatoïde, le syndrome anti-phospholipide, la maladie de Berger, le syndrome d'Evans, la rectocolite hémorragique, la glomérulonéphrite allergique liée aux anticorps, la granulocytopénie, le syndrome de Goodpasture, l'hépatite, le purpura de Henoch-Schönlein, la vascularite d'hypersensibilité, l'anémie immuno-hémolytique, le purpura thrombopénique idiopathique, le syndrome de Kawasaki, la conjonctive allergique, le lupus érythémateux, le lymphome de Mantelzell, la neutropénie, la sclérose latérale non familiale, la maladie de Crohn, la sclérose en plaques, la myasténie grave, le syndrome myéloplastique, l'ostéoporose, les maladies ostéolytiques, l'ostéopénie, le psoriasis, le syndrome de Sjögren, la sclérodermie, le lymphome à cellules T, l'urticaire/ l'angio-oedème, la granulomatose de Wegener et la maladie coeliaque.

6. Utilisation selon la revendication 5, dans laquelle la maladie est choisie dans le groupe comprenant l'asthme, la BPCO, la rhinite allergique, le syndrome de détresse respiratoire de l'adulte, la bronchite, la dermatite allergique, la dermatite de contact, le purpura thrombopénique idiopathique, l'arthrite rhumatoïde et la rhinoconjonctivite allergique.

7. Utilisation de composés selon l'une des revendications 5 ou 6, dans laquelle la maladie est choisie dans le groupe comprenant l'asthme, la BPCO, la
rhinite allergique, la dermatite allergique et l'arthrite rhumatoïde.

8. Formulations pharmaceutiques **caractérisées par** la teneur en un ou plusieurs composés de formule 1 selon l'une des revendications 1 à 3.

9. Formulations pharmaceutiques, **caractérisées par** la teneur en un ou plusieurs composés de formule 1 selon l'une des revendications 1 à 3, en combinaison avec une substance active choisie dans le groupe comprenant les bêtamimétiques, les corticoïdes, les inhibiteurs de PDE4, les inhibiteurs de l'EGFR et les antagonistes du LTD4, les inhibiteurs de CCR3, les inhibiteurs de l'iNOS et les inhibiteurs de SYK.

10. Composés choisis dans le groupe comprenant 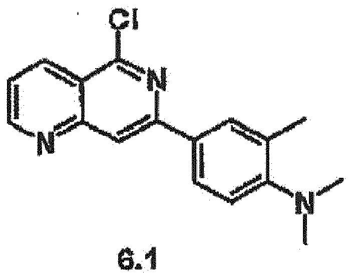 et
